(19) Europäisches Patentamt
European Patent Office
Office européen des brevets

(11) EP 2 601 940 A1

(12) EUROPEAN PATENT APPLICATION

(43) Date of publication:
**12.06.2013 Bulletin 2013/24**

(21) Application number: **11382377.7**

(22) Date of filing: **05.12.2011**

(51) Int Cl.:
***A61K 31/00*** *(2006.01)* ***A61K 31/445*** *(2006.01)*
***A61K 31/455*** *(2006.01)* *A61P 1/04 (2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **LACER, S.A.**
**08025 Barcelona (ES)**

(72) Inventors:
- **Mourelle Mancini, Marisabel**
  **E-08028 Barcelona (ES)**
- **Del Castillo Nieto, Juan Carlos**
  **E-08025 Barcelona (ES)**

(74) Representative: **ABG Patentes, S.L.**
**Avenida de Burgos, 16D**
**Edificio Euromor**
**28036 Madrid (ES)**

(54) **(4-mercapto-piperidin-4-yl)-aminoacetic acid derivatives, pharmaceutical compositions and uses thereof**

(57) The invention provides (4-mercapto-piperidin-4-yl)-aminoacetic acid derivatives of formula (I), or a pharmaceutically acceptable salt or stereoisomer thereof, for use as a medicament, preferably for use in the treatment and/or prophylaxis of gastrointestinal ulcer, as well as pharmaceutical compositions comprising said thiol derivatives, or a pharmaceutically acceptable salt or stereoisomer thereof, and one or more pharmaceutically acceptable carriers.

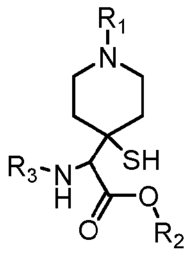

(I)

EP 2 601 940 A1

## Description

### FIELD OF THE INVENTION

The present invention relates to (4-mercapto-piperidin-4-yl)-aminoacetic acid derivatives useful for the preparation of medicaments.

### BACKGROUND OF THE INVENTION

Nonspecific ulcers in the upper gastrointestinal tract (stomach and duodenum), commonly but inaccurately referred to as "peptic ulcers," affect about 10% of the population of the United States at least once in a lifetime [Sutton D. R. and Donaldson M., Gastroenterology 69:166-174 (1975)]. Drug-induced (iatrogenic) ulcers are even more common.

A large proportion of chronic users of aspirin and nonsteroidal anti-inflammatory drugs (e.g., patients with rheumatoid arthritis and osteoarthritis, and healthy people seeking prevention of disease) are affected by drug-induced ulcers in a dose- and time-dependent manner [Lanza, F. L., et al., Amer. J. Gastroenterol. 80:767-769 (1985); Lorenz, R. L., et al., Lancet 1:1261-1264 (1984)].

Ulcer inducing agents are also referred to as "ulcerogenic" agents. "Ulcer disease," which is a more accurate designation than "peptic ulcer," is considered a mass disorder because, it affects a large segment of the population. Similarly, the pathophysiology of ulcer disease is poorly understood. It is now clear that ulcer disease is a complex disorder that is multifactorial or pluricausal in origin [Brooks, F. P., in Peptic Ulcer Disease: Contemporary Issues in Gastroenterology, Brooks, F. P., et al., eds., Churchill-Livingston, New York, 1985, 145-151; Szabo, S., Laboratory Investigations 51:121-147 (1984)].

The multifactorial etiology and pathogenesis imply that it is unrealistic to expect a complete healing or a preventive effect from highly specific drugs that affect only one component in this complex chain of events. Thus, it is not surprising that after cessation of treatment with even the most potent of the current antisecretory agents, the H2-receptor antagonists such as cimetidine, the recurrence rate of chronic duodenal ulcers is 40-60% a year [Thomas, J. M., et al., Clin. Gastroenterol. 13:501-541 (1984)].

It has been reported that "Novel drugs which affect more than one element in the pathogenesis of ulcer disease are thus realistically expected to have a more profound effect on ulcer healing and recurrence than presently available antiulcer drugs. Future research must address the different etiologies of gastric and duodenal ulcers and other acid-peptic conditions, as well as attempting to cure the disease, rather than simply heal the ulcer." [Garner, A., Scand. J. Gastroenterol. 21, Suppl. 125:203-210 (1986)].

"Gastric cytoprotection", a recently developed concept in ulcer prevention, is focused on the prevention of acute mucosal lesions without decreasing gastric acidity. Based on this concept, new compounds have been developed to provide protection for the gastric mucosa. As originally defined, gastric "cytoprotection" or "gastroprotection" referred to several specific modalities used to prevent hemorrhagic gastric erosions without inhibiting acid secretion.

Examples of these specific modalities include: the prostaglandins; sulfhydryl group-containing drugs that protect animals from ethanol-induced gastric erosions; and certain other agents [Miller, T. A., Amer. J. Physiol. 245:G601-623 (1983); Szabo, S., et al., Science 214:200-202 (1981)]. Early vascular damage is an important event in the process leading to hemorrhagic mucosal injury, and vascular lesions have been shown to be early pathogenetic factors in gastric hemorrhagic erosions [Leung et al., Gastroenterology 88:1948-1953 (1985); Szabo et al., Gastroenterology 88:228-236 (1985)]. Therefore, the prevention of chemically induced vascular endothelial damage in the gastric mucosa may be a major mechanism of gastric cytoprotection.

In the context of the present invention drugs capable of providing gastric cytoprotection are antiulcer agents, i.e., drugs that exert gastroprotective or enteroprotective effects without suppressing normal functions such as gastric acid and pepsin secretion. The subject matter of the present invention relates to the use of known (4-mercapto-piperidin-4-yl)-aminoacetic acid derivatives as gastric cytoprotectants, useful in treatment and/or prophylaxis of gastrointestinal ulcer.

International patent application WO 2008/080534, in the name of the applicant, describes S-nitrosothiols as NO releasing agents which are capable of eliciting a vasodilating activity and also inhibion effect of the aggregation of platelets. This document also discloses the (4-mercapto-piperidin-4-yl)-aminoacetic acid derivatives of the present invention, but only as synthetic intermediates in the synthesis of S-nitrosothiols. WO 2008/080534 does not provide any indication about a medical use of said intermediate (4-mercapto-piperidin-4-yl)-aminoacetic acid derivatives, much less on their use as antiulcer agents. The inventors have now surprisingly found that the thiol intermediates described in WO 2008/080534 are useful as medicaments to provide gastroprotection or enteroprotection in the treatment or prophylaxis of gastrointestinal ulcer.

## SUMMARY OF THE INVENTION

A first aspect of the invention is directed to (4-mercapto-piperidin-4-yl)-aminoacetic acid derivatives of formula (I),

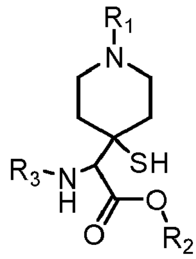

(I)

wherein
$R_1$ and $R_2$ are each independently selected from the group consisting of alkyl, alkenyl, cycloalkyl, alkynyl, aryl, aralkyl and heterocyclyl;
$R_3$ is selected from the group consisting of hydrogen, alkyl, cycloalkyl, alkenyl, alkynyl, heterocyclyl, $-C(=O)R_4$ and $-S(=O)_2R_4$, wherein $R_4$ is selected from the group consisting of hydrogen, alkyl, alkenyl, cycloalkyl, alkynyl, aryl, aralkyl and heterocyclyl;
wherein $R_4$ is optionally mono-, di- or trisubstituted by a group independently selected from alkyl, alkyloxy, halogen and nitro;
or a pharmaceutically acceptable salt or stereoisomer thereof, for use as a medicament, more particularly as a medicament useful in the treatment and/or prophylaxis of gastrointestinal ulcer.

A second aspect of the invention relates to a pharmaceutical composition comprising a compound of formula (I) as defined above, or a pharmaceutically acceptable salt or stereoisomer thereof, and one or more pharmaceutically suitable excipients.

## DETAILED DESCRIPTION OF THE INVENTION

Compounds of formula (I)

A first aspect of the present invention relates to (4-mercapto-piperidin-4-yl)-aminoacetic acid derivatives of formula (I), as defined above, or a pharmaceutically acceptable salt or stereoisomer thereof for use as a medicament.

In one embodiment, the present invention provides a compound of formula (I) as previously defined, wherein $R_3$ is selected from the group consisting of $-C(=O)R_4$ and $-S(=O)_2R_4$, and wherein $R_4$ is as defined above. Preferably, $R_4$ is selected from the group consisting of hydrogen; alkyl optionally mono-, di-, or trisubstituted by halogen; aryl optionally mono-, di-, or trisubstituted by a group independently selected from alkyl, alkyloxy, halogen and nitro; and heterocyclyl. More preferably $R_4$ is selected from the group consisting of hydrogen; methyl optionally mono-, di-, or trisubstituted by halogen hexyl, phenyl optionally mono- or disubstituted by halogen, methyl, methyloxy or nitro; and pyridyl. Even more preferably, $R_4$ is selected from the group consisting of hydrogen; methyl optionally mono-, di-, or trisubstituted by halogen; and phenyl optionally mono- or disubstituted by halogen or nitro.

In another embodiment, the present invention provides a compound of formula (I) as defined above, wherein $R_1$ and $R_2$ are each independently selected form the group consisting of alkyl and aralkyl. Preferably, $R_1$ and $R_2$ are each independently selected from the group consisting of methyl, ethyl and benzyl. More preferably, $R_1$ is methyl or benzyl. More preferably, $R_2$ is ethyl.

In yet another embodiment, the present invention provides a compound of formula (I), selected from the group consisting of:

- ethyl (R,S)-2-(4-mercapto-1-methylpiperidin-4-yl)-2-(phenyl sulfonamido)acetate;
- ethyl (R,S)-2-(4-mercapto-1-methylpiperidin-4-yl)-2-(4-nitrophenyl sulfonamido) acetate;
- ethyl (R,S)-2-(3-chlorophenylsulfonamido)-2-(4-mercapto-1-methyl piperidin-4-yl)acetate;
- ethyl (R,S)-2-(2-fluorophenylsulfonamido)-2-(4-mercapto-1-methyl piperidin-4-yl)acetate;
- ethyl (R,S)-2-(2,4-difluorophenylsulfonamido)-2-(4-mercapto-1-methyl piperidin-4-yl)acetate;
- ethyl (R,S)-2-acetamido-2-(1-benzyl-4-mercaptopiperidin-4-yl)acetate;
- ethyl (R,S)-2-(1-benzyl-4-mercaptopiperidin-4-yl)-2-formamidoacetate;

or a pharmaceutically acceptable salt or stereoisomer thereof.

The invention also provides salts of compounds of the invention. For instance, pharmaceutically acceptable salts of compounds provided herein may be acid addition salts, base addition salts or metallic salts, and they can be synthesized from the parent compound which contains a basic or an acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, ammonium, and organic alkali salts such as, for example, ethylenediamine, ethanolamine, N,N-dialkylenethanolamine, triethanolamine, glucamine and basic aminoacids salts. Examples of the metallic salts include, for example, sodium, potassium, calcium, magnesium, aluminium and lithium salts.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

It will be readily apparent to those skilled in the art that the scope of the present invention also encompasses salts which are not pharmaceutically acceptable as possible means for obtaining pharmaceutically acceptable salts.

It will be immediately apparent to the skilled person that the present invention encompasses all possible stereoisomers of the compounds described herein. A stereoisomer is understood by the skilled person as a compound made up of the same atoms bonded by the same sequence of bonds but having different three-dimensional structures which are not interchangeable. All stereoisomers of the compounds of this invention are contemplated either alone or as mixtures thereof, such as racemates, enantiomers, or diastereomers. When diastereomeric or enantiomeric mixtures are prepared, they can be separated by conventional methods, for example, chromatographic or functional crystallization.

<u>Synthesis of the compounds of formula (I) and intermediates of the same</u>

There are several alternative methods for the synthesis of compounds of formula (I), wherein $R_1$, $R_2$ and $R_3$ are as defined above. Scheme I below shows two methods for the synthesis of said compounds.

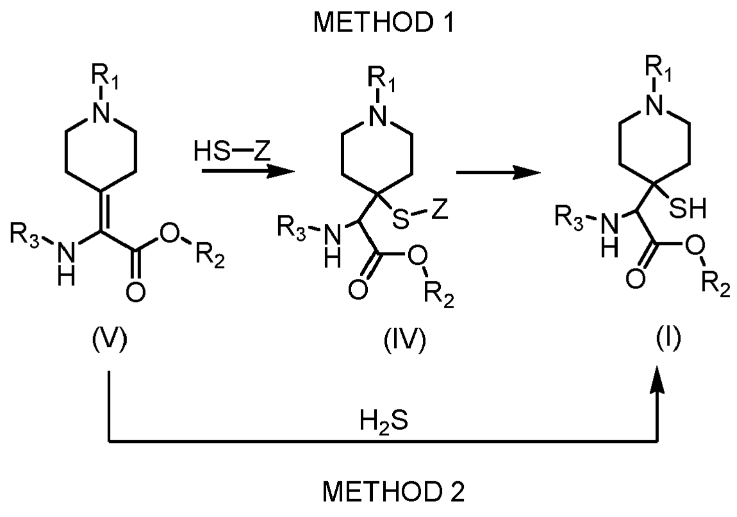


Scheme I

In the first method compounds of formula (I), wherein $R_1$, $R_2$ and $R_3$ are as defined above, may be obtained by removal of the Z group from a compound of formula (IV)

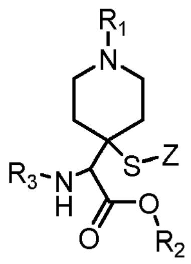

(IV)

wherein

$R_1$, $R_2$ and $R_3$, are as defined above for formula (I), and Z is selected from the group consisting of unsubstituted alkyl, alkenyl, $C_7$-$C_{20}$ aralkyl, -Si(R')$_3$, —C(=O)R', wherein R' is selected from the group consisting of alkyl, alkenyl, alkynyl, aryl and aralkyl.

Z is usually selected so as to be easily cleaved at this stage of the synthesis. For example, when -S-Z is a thioether, such as methyl thioether, tert-butyl thioether, benzyl thioether, p-methoxybenzyl thioether, 3,4-dimethoxybenzyl thioether, trityl thioether; allyl thioether; when -S-Z form silyl derivatives wherein Z is —Si(R')$_3$ such as trimethylsilyl (also represented as "TMS"), triethylsilyl, tert-butyldimethylsilyl (also **represented as** "TBDMSO"), **tert**-butyldiphenylsilyl, tri-isopropylsilyl, diethylisopropylsilyl, thexyldimethylsilyl, triphenylsilyl, di-tert-butylmethylsilyl; or when -S-Z form thioesters such as acetate thioester, benzoate thioester; pivalate thioester; methoxyacetate thioester; chloroacetate thioester; levulinate thioester. Further conditions under which said groups may be removed, can be found in T.W. Greene and P.G.M. Wuts en "Protective Groups in Organic Synthesis", 3a Edicción, Wiley-Interscience, pages 454-493.

Preferably, Z is a $C_7$-$C_{20}$ aralkyl group (e.g. p-methoxybenzyl) which may be removed under standard conditions (see T.W. Greene et al.), such as acidic conditions (e.g. trifluoroacetic acid) or hydrogenolysis.

In turn, the compounds of formula (IV) may be obtained by the addition of a compound of formula HS-Z, wherein Z is as defined above, in the presence of a base, to a compound of formula (V)

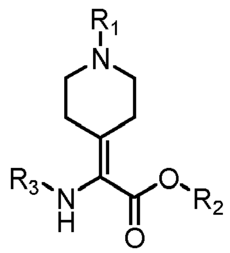

(V)

wherein

$R_1$, $R_2$ and $R_3$, are as defined in formula (I).

Conditions for this kind of reactions can be found in Nelson C.F. Yim et al., J. Org. Chem., 1988, 53, 4605-7 and C. Freeman Stanfield et al., Synthetic Communications, 1988, 18(5), 531-43.

The second method depicted above in Scheme I for the synthesis of the compounds of formula (I) comprisesthe addition of $SH_2$ to a compound of formula (V)

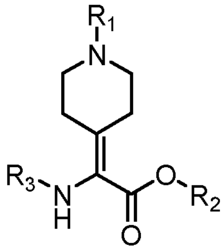

(V)

wherein

$R_1$, $R_2$ and $R_3$, are as defined in above, to obtain a compound of formula (I).

Thus, with this method it is possible to provide the compounds of formula (I) in a single step from the compounds of formula (V).

The compounds of formula (V), wherein $R_3$ is a formyl group, which are the precursor compounds for both synthetic methods described above, are readily available by reaction of ketones of formula (VI)

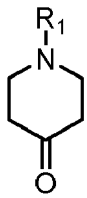

(VI)

wherein $R_1$, is as defined in formula (I);

with alkylisocyanoacetates of formula (VII)

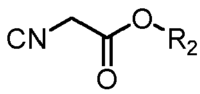

(VII)

wherein $R_2$ is as defined in formula (I).

The conditions for this reaction may be found in DE 2801849 and Nunami Kenichi et al., J. Chem. Soc. Perkin Trans. 1, 1979, 9, 2224-9.

The resulting compounds of this reaction are compounds of formula (V), wherein $R_3$ is a formyl group. Thus, if the compound to be obtained has a $R_3$ different from a formyl group, the appropriate functionality must be introduced in $R_3$ by methods known to the skilled person prior to the formation of the compounds of formula (I), or prior to the formation of the compounds (IV). The election of the precise stage at which to introduce or modify said $R_3$ groups will depend on the compatibility of the different functional groups in the molecule. Said incompatibilities are a matter of common general knowledge or routine experimentation for the skilled person.

In the cases where the introduction of the $R_3$ functionality is carried out prior to the formation of the compound of formula (IV), it is possible, for example, to prepare a compound of formula (Va) (wherein $R_3$ in the compound of formula (V) is a formyl group) from a compound of formula (VI) and a compound of formula (VII) as described above, and then remove the formyl group (by hydrolysis), yielding a compound of formula (Vb) (wherein $R_3$ in the compound of formula (V) is a hydrogen atom). One alternative, which is shown bellow in path A of scheme II, is introducing the $R_3$ functionality (different form hydrogen) at this stage, i.e. after removal of the formyl group and prior to the formation of the compound of formula (IV), followed by the conversion of said compound of formula (V) having an $R_3$ group different from hydrogen to a compound of formula (IV) having also an $R_3$ group different from hydrogen. Another alternative, which is shown bellow in path B of scheme II, is first carrying out the conversion of the compound of formula (Vb), to a compound of formula (IVb) (wherein $R_3$ in the compound of formula (IV) is a hydrogen atom), followed by the introduction of $R_3$

(different from hydrogen) yielding a compound of formula (IV) having an $R_3$ different from hydrogen In both alternatives, the target compound of formula (I) is obtained from said intermediate compound of formula (IV) having an $R_3$ different from hydrogen.

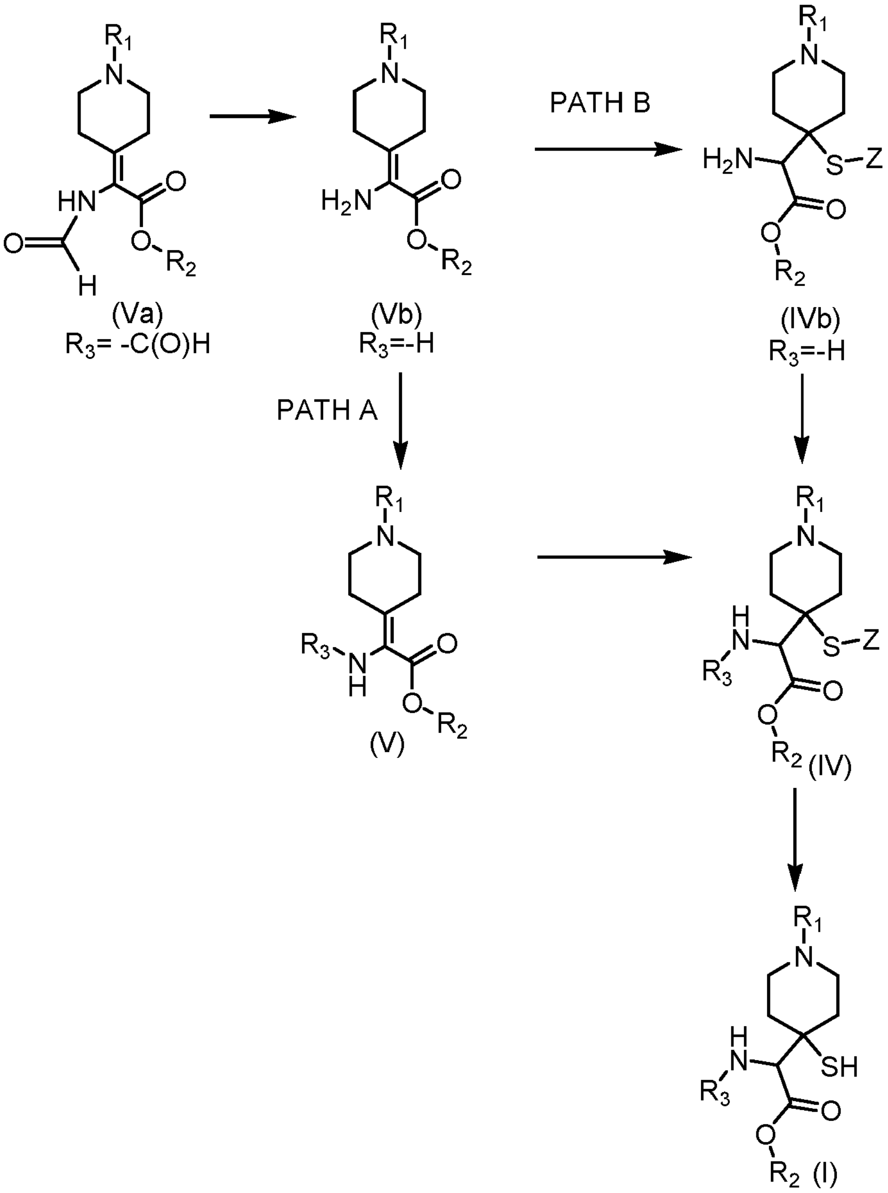


Scheme II

Another alternative is introducing the $R_3$ functionality prior to the formation of a compound of formula (I) but after the formation of a compound of formula (IV). Then, a compound of formula (Va) (wherein $R_3$ in the compound of formula (V) is a formyl group) may be prepared from a compound of formula (VI) and a compound of formula (VII), as explained above. Then, transform said compound of formula (Va) into a compound of formula (IVa) (wherein $R_3$ in the compound of formula (IV) is formyl), and then remove the Z group from said compound of formula (IVa) to form a compound of formula (Ia) (wherein $R_3$ in the compound of formula (I) is formyl). Said compound of formula (Ia) may be used to prepare another compound of formula (I) having an $R_3$ different from formyl. This alternative is shown bellow in scheme III. Alternatively, the $R_3$ functionality may be introduced after the formation of the compound of formula (IVa), yielding a compound of formula (IV), which is then used to prepare a compound of formula (I). This alternative is also shown below in scheme III.

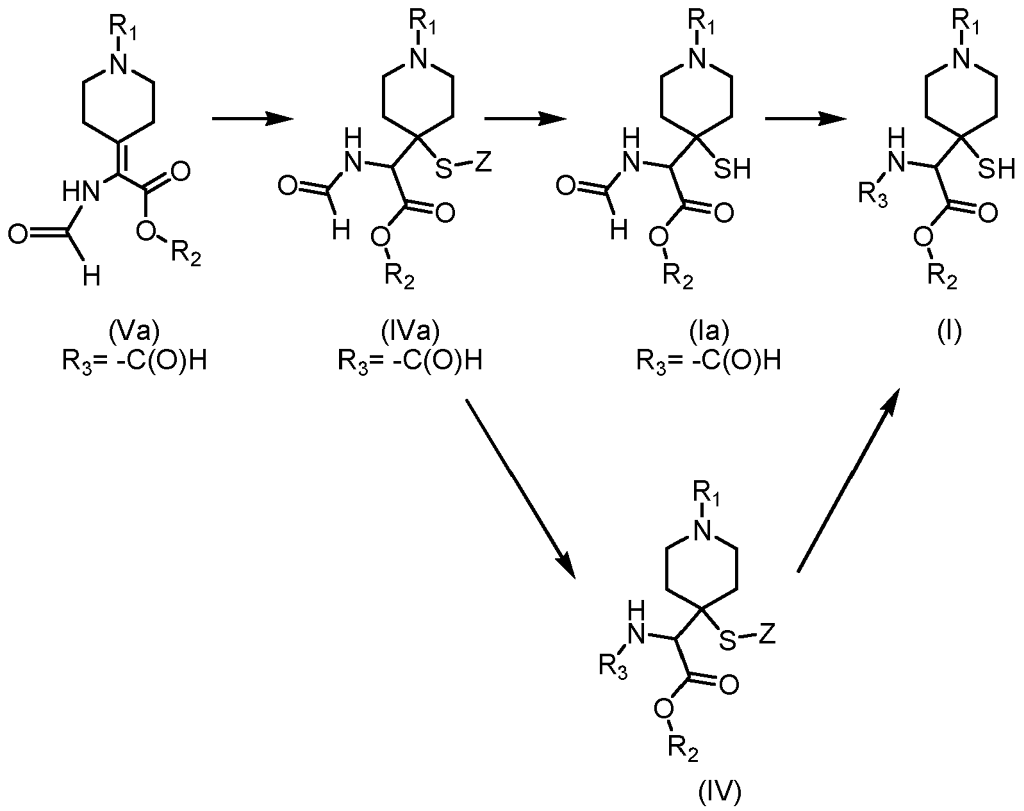

(Va) $R_3$= -C(O)H

(IVa) $R_3$= -C(O)H

(Ia) $R_3$= -C(O)H

(I)

(IV)

Scheme III

Other possible combinations will be readily apparent to the skilled person.

Uses of the compounds of the invention

As stated above, in the first aspect, the invention is directed to the compounds of formula (I) as defined above, for use as a medicament.

In one embodiment, the present invention provides a compound of formula (I) as defined above, or a pharmaceutically acceptable salt or stereoisomer thereof, for use in the treatment and/or prophylaxix of gastrointestinal ulcer.

The term "treatment and/or prophylaxis" in the context of this specification means administration of a compound or formulation according to the invention to preserve health in a patient suffering or in risk of suffering a gastrointestinal ulcer. Said terms also include administration of a compound or formulation according to the invention to prevent, ameliorate or eliminate one or more symptoms associated with a gastrointestinal ulcer.

According to a further aspect, the present invention is directed to a pharmaceutical composition comprising a compound of the invention of formula (I) as defined above and a pharmaceutically acceptable excipient.

The term "excipient" refers to a diluent, adjuvant, carrier, or vehicle with which the active ingredient is administered. Such pharmaceutical excipients can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions, also buffers, isotonic agents or agents capable increasing solubility. Suitable pharmaceutical carriers are described in "Remington's Pharmaceutical Sciences" by E.W. Martin or "Tratado de Farmacia Galénica", C. Faulí i Trillo, Luzán 5, S.A. de Ediciones, 1993.

Preferably the pharmaceutical compositions are aqueous or non-aqueous compositions which comprise a compound of the invention of formula (I) as defined above and a pharmaceutically acceptable excipient other than water.

The pharmaceutical composition of the invention may be administered in the form of different preparations. Non limiting examples of pharmaceutical compositions of the invention are preparations for oral administration, i.e. tablets, capsules, syrups or suspensions. Also, the pharmaceutical compositions of the invention may include topical compositions, i.e. creams, ointments or pastes, or transdermic preparations such as patches or plasters. The pharmaceutical composition of the invention may also be prepared for rectal administration, i.e. rectal gel or rectal capsule.

In a preferred embodiment the pharmaceutical compositions are in oral form, either solid or liquid. Suitable

dose forms for oral administration may be tablets, capsules, syrops or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycolate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

Generally an effective administered amount of a compound used in the invention will depend on the relative efficacy of the compound chosen, the severity of the disorder being treated, or the age, weight or mode of administration. However, active compounds will typically be administered once or more times a day, for example 1, 2, 3 or 4 times daily, with typical total daily doses in the range of from 0.1 to 500 mg/kg/day.

The compounds used in the present invention may also be administered with other drugs to provide a combination therapy. The other drugs may form part of the same composition, or be provided as a separate composition for administration at the same time or at different time.

<u>Definitions</u>

In the definitions of the compounds described herein the following terms have the meaning indicated:

"Alkyl" refers to a straight or branched hydrocarbon chain radical consisting of carbon and hydrogen atoms, containing no unsaturation, having one to twelve carbon atoms, preferably one to eight carbon atoms, more preferably one to six carbon atoms, and which is attached to the rest of the molecule by a single bond, e. g., methyl, ethyl, n-propyl, i-propyl, n-butyl, t-butyl, n-pentyl, etc.

"Alkenyl" refers to a straight or branched hydrocarbon chain radical consisting of carbon and hydrogen atoms, containing at least one carbon-carbon double bond, having two to twelve carbon atoms, preferably two to eight carbon atoms, more preferably two to six carbon atoms, and which is attached to the rest of the molecule by a single bond, such as vinyl, allyl, 1.-propenyl, 2-propenyl, 1- butenyl, 2-butenyl, and 3-butenyl.

"Cycloalkyl" refers to a saturated carbocyclic ring consisting of carbon and hydrogen atoms having from three to eight carbon atoms, preferably three to six carbon atoms. Suitable cycloalkyl groups include, but are not limited to cycloalkyl groups such as cyclopropyl. cyclobutyl, cyclopentyl or cyclohexyl.

"Alkynyl" refers to a straight or branched hydrocarbon chain radical consisting of carbon and hydrogen atoms, containing at least one carbon-carbon triple bond, conjugated of not, having two to twelve, preferably two to eight, more preferably two to six carbon atoms, and which is attached to the rest of the molecule by a single bond, such as —CCH, $-CH_2CCH$, $-CCCH_3$, $-CH_2CCCH_3$.

"Aryl" refers to an aromatic hydrocarbon radical having six to ten carbon atoms in the ring, such as phenyl or naphthyl.

"Aralkyl" refers to an aryl group as defined above linked to the rest of the molecule by an alkyl group as defined above, such as benzyl and phenethyl.

"Heterocyclyl" refers to a stable 3- to 15- membered ring which consists of carbon and hydrogen atoms, and from one to five heteroatoms selected from the group consisting of nitrogen, oxygen, and sulphur, preferably a 4-to 8-membered ring with one, two, three or four heteroatoms, more preferably a 5-or 6-membered ring with one, two or three heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, pyridine, purine, quinoline, thiadiazole, tetrahydrofuran.

The term "stable", as used herein, refers to compounds that possess stability sufficient to allow manufacture thereof or that maintain their chemical integrity for a sufficient period of time to be detected and or to be useful for the purposes detailed herein.

The term "halogen" or "halo" refers to chlorine, bromine, fluorine, and iodine.

The term "alkoxy" or "alkyloxy" refers to an alkyl group as defined above linked to an oxygen atom, such as methoxy, ethoxy, n-propoxy, isopropoxy, n-butoxy, isobutoxy, sec-butoxy, and tert-butoxy.

In the context of the present invention, when a group is said to be mono-, di-, or trisubstituted it is to be understood that 1, 2 or 3 hydrogen atoms, respectively, in said group are replaced by 1, 2 or 3 substituents, respectively.

**Experimental section**

The examples set forth in this description detail the suitable processes for obtaining several compounds which can be assigned to formula (I). In view of said examples, it is evident and direct for a person skilled in the art to obtain the compounds which are not explicitly exemplified, by means of applying modifications of the methods set forth, characteristic of the common general knowledge of the persons skilled in the art.

Consequently, the examples set forth below must not be interpreted as limiting the scope of the present invention, but rather as an additional and more detailed explanation which guides the person skilled in the art to a deeper understanding of the invention.

The following abbreviations are used in the following examples:

| | |
|---|---|
| AcOEt | Ethyl acetate |
| AcOH | Acetic acid |
| DMSO-$d_6$ | Hexadeuterodimethyl sulfoxide |
| EtOH | Ethanol |
| $Et_2O$ | Diethyl ether |
| HPLC | high pressure liquid chromatography |
| IPA | Isopropyl alcohol |
| RT | Room temperature |
| THF | Tetrahydrofuran |
| TLC | Thin layer chromatography |

The nuclear magnetic resonance spectra have been carried out in a Varian Gemini-200 apparatus.

The $^1H$—NMR spectra indicate the working frequency and the solvent used to make the spectrum. The position of the signals is indicated in $\delta$ (ppm), using the signal of the protons of the solvent as reference. The reference values are 7.24 ppm for chloroform and 2.49 ppm for deuterated dimethyl sulfoxide. Within brackets are indicated the number of protons corresponding to each signal measured by electronic integration and the type of signal using the following abbreviations: s (singlet), d (doublet), t (triplet), q (quadruplet), s.b. (signal broad). The signal assignation is indicated in some cases.

The $^{13}C$-NMR spectra indicate the working frequency and the solvent used to make the spectrum. The position of the signals is indicated in $\delta$ (ppm), using the central signal of the solvent as reference. The reference values are 77.00 ppm for chloroform and 39.50 ppm for deuterated dimethylsulfoxide.

When analyses by HPLC are carried out to determine the global purity or stability of some of the samples, the determinations are performed by HPLC using an HPLC apparatus equipped with an UV detector (201 nm). Chromatographic separation is performed using an RP18 column, 150 x 4.6 mm, 5$\mu$m, at 30 °C, with a gradient method from 90 % to 10 % of A in 30 min, where A is 5 mM ammonium formate at pH 3 and the organic phase is acetonitrile. The flow rate is set at 1 ml/min, and the compartment of samples is set at 4 °C. Samples are prepared at 1 mg/ml, in acetonitrile / ammonium formate 5 mM at pH 3 (1/2).

**A) SYNTESIS OF THE RELEVANT COMPOUNDS**

**Example 1.**

**1a).- Synthesis of Ethyl formylamino-(1-methyl-piperidin-4-ilydene)-acetate**

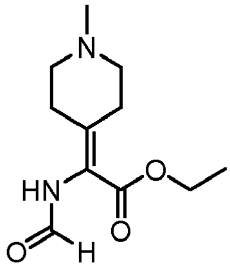

180 g (1.6 moles) of potassium tert-butoxide and 1L of dry THF were added to a 6L three-neck flask, equipped with a condenser, a thermometer and a calcium chloride tube. The mixture was cooled with an ice bath and 181 ml (1.6 moles) of ethyl isocyanoacetate dissolved in 180 ml of dry THF were added dropwise, keeping the temperature below 15°C. After the addition, 187 ml (1.6 moles) of 1-methyl-4-piperidone dissolved in 187 ml of dry THF were added dropwise, keeping the temperature below 15°C. After the addition, the mixture was allowed to reach room temperature and it was stirred for 30 minutes. 900 ml of a 1/1 $AcOH/H_2O$ solution was added, controlling that the temperature did not exceed 45°C. After the addition, the stirring was maintained for 1 hour. The THF was eliminated under vacuum and 1L of water was added. 350 g of sodium carbonate were added until reaching a pH between 8 and 9 and it was extracted with 5x600 ml of dichloromethane. The organic phase was dried and concentrated to afford 248 g of a dark brown solid which was recrystallized with 1.4 L of a 1/1 toluene/cyclohexane mixture to yield 167 g of the title compound as a light brown solid (Yield: 46%).

**$^1$H -NMR ($D_2O$, 200 MHz):** 7.80 (s, 1H, CHO); 3.93 (q, 2H, $OCH_2$); 2.50-2.10 (m, 8H, piperidine); 1.93 (s, 3H, $NCH_3$); 0.95 (t, 3H, $CH_3$)

**$^{13}$C-NMR ($D_2O$, 200 MHz):** 165.81 (C=O); 163.12 (C=O); 146.88 (C=C); 117.69 (C=C); 61.94 ($OCH_2$); 54.35 and 53.98 ($NCH_2$); 43.53 ($NCH_3$); 29.08 and 28.41 ($CH_2$); 12.76 ($CH_3$)

**1b).- Synthesis of Ethyl (R,S)-formylamino-[1-methyl-4-(4-methoxy-benzylsulfanyl)-piperidin-4-yl]-acetate**

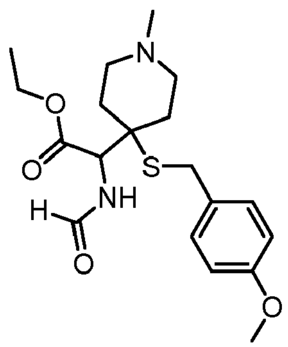

16.09 g of 55% sodium hydride in mineral oil (0.367 moles) and 300 ml of toluene were added to a 1L three-neck flask, equipped with a condenser and a thermometer. The mixture was cooled to 10°C and 56.8 ml (0.367 moles) of 4-methoxybenzylthiol dissolved in 200 ml of toluene were quickly added and the mixture was stirred at this temperature for 5 minutes. 83.0 g (0.367 moles) of ethyl formylamino-(1-methyl-piperidin-4-ilydene)-acetate, product obtained in (la), were added in portions, preventing the temperature from exceeding 35°C. After the addition, the mixture was maintained at RT for 3 hours. 370 ml of 2N hydrochloric acid were added and the phases were separated. The aqueous phase was washed with 2x300ml of toluene to eliminate impurities. The aqueous phase was taken to pH 10-11 with 2N NaOH and it was extracted with 4x300 ml of dichloromethane. The combined organic fractions were dried on sodium sulfate, filtered and concentrated. 125.7 g of the title compound were obtained as a completely pure amber oil (purity determined by TLC ($CH_2Cl_2/EtOH/NH_4OH$) and NMR). Yield: 90%

**$^1$H -NMR (DMSO-$d_6$, 200 MHz):** 8.70 (d, J=9.2Hz; 1H, NH); 8.11 (s, 1H, CHO); 7.21 (d, J=8.6Hz, 2H); 6.86 (d, J=8.6Hz, 2H); 4.74 (d, J=9.2Hz, 1H, CH); 4.16 (q, 2H, $OCH_2$); 3.72 (s, 3H, $OCH_3$); 3.63 (s, 2H, $SCH_2$); 2.60-2.20 (m, 4H, piperidine); 2.16 (s, 3H, $NCH_3$); 1.90-1.50 (m, 4H, piperidine); 1.24 (t, 3H, $CH_3$)

**$^{13}$C-NMR (DMSO-$d_6$, 200 MHz):** 169.50 (C=O); 161.23 (C=O); 158.30 (C-O); 130.26 (2C, CH); 128.67 (1C, $\underline{C}$-$CH_2$);113.85 (2C, CH); 60.81 (O$\underline{C}H_2$); 57.01 (1C, CH); 55.04 (1C, $OCH_3$); 50.30 and 50.08 (3C, 2 $NCH_2$ and C4piperidine); 45.74 ($NCH_3$); 30.70 and 30.22 (3C, $2CH_2$ and S-$CH_2$); 14.02 (1C, $CH_3$)

**1c).- Synthesis of Ethyl (R,S)-amino-[1-methyl-4-(4-methoxy-benzylsulfanyl)-piperidin-4-yl]-acetate**

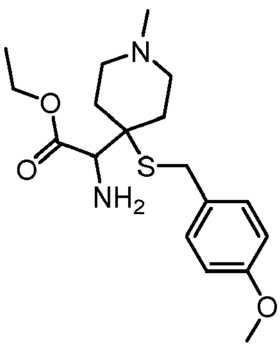

125.7 g (0.330 moles) of ethyl (R,S)-formylamino-[1-methyl-4-(4-methoxy-benzylsulfanyl)-piperidin-4-yl]-acetate and 300 ml of a 6M EtOH/HCl solution were added to a 1L flask and heated under reflux for 24 hours. The mixture was concentrated to dryness, the residue was dissolved in 700 ml of water and washed with 2x200 ml of AcOEt. The aqueous phase was basified with 30% $NH_4OH$ and extracted with 3x300 ml of dichloromethane. The organic phase was dried, filtered and concentrated to afford 109.3 g (Yield: 94%) of the title compound as a completely pure amber oil (determined by TLC ($CH_2Cl_2$/EtOH/$NH_4OH$) and NMR).

**$^1$H -NMR (DMSO-$d_6$, 200 MHz):** 7.21 (d, J=8.4Hz, 2H); 6.86 (d, J=8.4Hz, 2H); 4.12 (q, 2H, $OCH_2$); 4.10-3.90 (m, 1H, CH); 3.72 (s, 3H, $OCH_3$); 3.60 (s, 2H, $SCH_2$); 2.50 (s.b, 2H, $NH_2$); 2.40-2.20 (m, 2H, piperidine); 2.15 (s, 3H, $NCH_3$); 2.10-1.50 (m, 6H, piperidine); 1.23 (t, 3H, $CH_3$)

**$^{13}$C-NMR (DMSO-$d_6$, 200 MHz):** 173.32 (C=O); 158.15 (C-O); 130.11 (2C, CH); 129.36 (1C, <u>C</u>-$CH_2$); 113.76 (2C, CH); 61.82 (1C, CH); 59.91 (O<u>C</u>$H_2$); 54.99 (1C, $OCH_3$); 51.23 and 50.60 ((3C, 2 $NCH_2$ and C4piperidine); 45.90 ($NCH_3$); 30.35, 30.21 and 30.11 (3C, $2CH_2$ and S-$CH_2$); 14.09 (1C, $CH_3$)

**1d).- Synthesis of Ethyl (R,S)-acetylamino-[1-methyl-4-(4-methoxy-benzylsulfanyl)-piperidin-4-yl]-acetate**

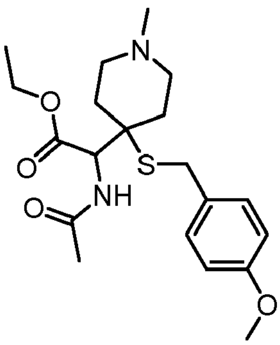

18 g (50 mmol) of ethyl (R,S)-amino-[1-methyl-4-(4-methoxy-benzylsulfanyl)-piperidin-4-yl]-acetate, product obtained in 1c, and 300 ml of dry THF were added to a 1L flask. The mixture was cooled to 0°C and 7.12 ml (50 mmol) of triethylamine were added. Then, 3.64 ml (50 mmol) of acetyl chloride in 10 ml of dry THF were added dropwise. The mixture was stirred for 2 hours at 0°C, concentrated to dryness and the residue was dissolved in 100 ml of dichloromethane and washed with 2x50 ml of a 5% sodium bicarbonate solution. The organic phase was dried, filtered and concentrated. 19.37g (Yield: 96%) of the title compound were obtained as an amber oil which crystallizes on standing.

**$^1$H -NMR ($CDCl_3$, 200 MHz):** 7.21 (d, J=8.8Hz, 2H); 6.82 (d, J=8.8Hz, 2H); 6.32 (d, J=9Hz, NH); 4.78 (d, J=9Hz, 1H, CH); 4.21 (q, 2H, $OCH_2$); 3.77 (s, 3H, $OCH_3$); 3.61-3.58 (m, 2H, $SCH_2$); 2.70-2.40 (m, 4H, piperidine); 2.28 (s, 3H, $NCH_3$); 2.00 (s, 3H, $COCH_3$); 2.10-1.60 (m, 4H, piperidine); 1.30 (t, 3H, $CH_3$)

**$^{13}$C-NMR ($CDCl_3$, 200 MHz):** 173.40 (C=O); 170.05 (C=O); 158.92 (C-O); 130.38 (2C, CH); 128.78 (1C, <u>C</u>-$CH_2$); 114.15 (2C, CH); 61.64 (O<u>C</u>$H_2$); 58.56 (1C, CH); 55.38 (1C, $OCH_3$); 50.91 ((3C, 2 $NCH_2$ and C4piperidine); 46.15 ($NCH_3$); 32.97, 32.05 and 31.27 (3C, $2CH_2$ and S-$CH_2$); 23.41(1C, CO-<u>C</u>$H_3$); 14.27 (1C, $CH_3$)

**1e).- Synthesis of (R,S)-4-(Acetylamino-ethoxycarbonyl-methyl)-4-mercapto-1-**methyl-piperidinium trifluoroacetate

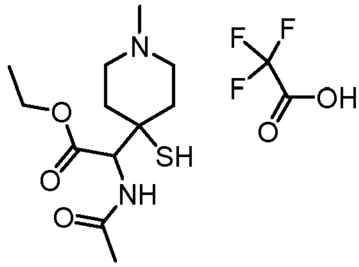

1.97 g (5 mmol) of the product obtained in 1d and 5 ml of trifluoroacetic acid were added to a 50 ml flask and heated under reflux for 20 hours. The mixture was allowed to cool and 20 ml of water were added and washed with 3x50 ml of AcOEt. The aqueous phase was concentrated to dryness by azeotropically distilling the remains of water with IPA. The residue was treated with $Et_2O$ with stirring to afford a white solid, which is filtered and dried. 1.7 g of the title product were obtained (Yield: 88%)

**$^1$H -NMR (DMSO-$d_6$, 200 MHz):** 10.60-9.80(s.b., 1H, $CO_2H$); 8.50-8.10 (s.b., 1H, NH); 4.52 (s.b., 1H, CH); 4.10 (q, 2H, $OCH_2$); 3.50-2.90 (m, 5H, 4Hpiperidine and SH); 2.77 (s, 3H, $NCH_3$); 2.10-1.60 (m, 7H, $COCH_3$ and 4Hpiperidine); 1.20 (t, 3H, $CH_3$)

**$^{13}$C-NMR (DMSO-$d_6$, 200 MHz):** 169.92 (C=O); 168.46 (C=O); 159.14 and 158.51 ($CO_2H$); 120.00 and 114.07 (CF3); 61.42 (1C, CH); 60.95 (O<u>C</u>$H_2$); 49.46 (2C, 2 $NCH_2$); 46.41 (1C, C4piperidine); 42.40 ($NCH_3$); 33.01 and 32.27 (2C, $2CH_2$); 22.26 (1C, $COCH_3$); 14.01 (1C, $CH_3$)

**1f).- Synthesis of (R,S)-ethyl 2-acetamido-2-(4-mercapto-1-methylpiperidin-4-yl)acetate hydrochloride**

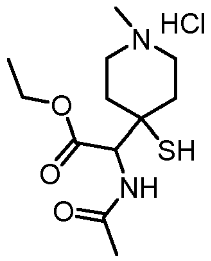

To 0.72g (1.85 mmol) of the product obtained in 1e, 10 ml (20mmol) of hydrochloric acid 2N were added. The solution was stirred during 5 minutes and a white solid crystallized. After addition of 10mL of water, the stirring was maintained during 15 min and then the mixture was filtered to yield 0.51g of the title product.

**$^{13}$C-NMR ($D_2O$, 200 MHz):** 173.83 (C=O); 169.47 (C=O); 62.48 (1C, CH); 61.61 (O<u>C</u>$H_2$); 49.65 and 49.41 (2C, 2 $NCH_2$); 45.35 (1C, C4piperidine); 42.44 ($NCH_3$); 33.33 and 32.40 (2C, $2CH_2$); 21.15 (1C, $COCH_3$); 12.76 (1C, $CH_3$)

By a process similar to that described in 1e followed by a process of deprotection similar to that described in 1f, the following products were obtained:

**Example 2. Ethyl (R,S) 2-heptanamido-2-(4-mercapto-1-methylpiperidin-4-yl) acetate 2,2,2-trifluoroacetate**

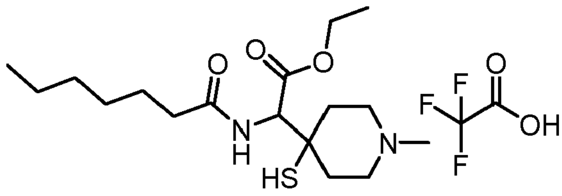

Yield last step: 61%. HPLC purity 93.2%

**$^1$H -NMR (DMSO-$d_6$, 200 MHz):** 10.40-10.20(s.b., 1H, $CO_2H$); 8.40-8.20 (s.b., 1H, NH); 4.58 (d, J=9.6Hz, 1H, CH); 4.10 (q, 2H, $OCH_2$); 3.89 (s.b. 2H); 3.60-3.10 (m., 2H); 2.78 (s, 3H, $NCH_3$); 2.20-1.80 (m, 6H); 1.48 (s.b., 2H, $CH_2$); 1.30-1.00 (m, 9H, $3CH_2$ + $OCH_2\underline{CH_3}$); 0.81 (t, 3H, $CH_3$)

**$^{13}$C-NMR (DMSO-$d_6$, 200 MHz):** 172.98 (C=O); 168.99 (C=O); 159.19 and 158.54 ($CO_2H$); 120.00 and 114.07 (CF3); 61.45 (1C, C4piperidine); 60.92 ($O\underline{C}H_2$); 49.50 (2C, $2NCH_2$); 46.35 (1C); 42.43 ($NCH_3$); 34.91 (1C, CO-$CH_2$), 33.02 and 32.05 (2C, $2CH_2$piperidine); 31.02 (1C); 28.31 (1C, $CH_2$); 25.25 (1C, $CH_2$); 22.06 (1C, $CH_2$); 13.99 and 13.92 (2C, 2CH3)

**Example 3. Ethyl (R,S)-2-benzamido-2-(4-mercapto-1-methylpiperidin-4-yl)acetate 2,2,2-trifluoroacetate**

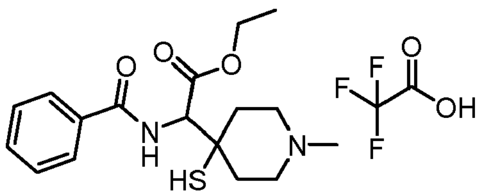

Yield last step: 33%.

**$^1$H -NMR ($CDCl_3$, 200 MHz):** 7.66 (d, J=7.6Hz, 1H); 7.56-7.43 (m, 3H); 7.17 (d, J=8.4Hz, 1H, NH); 5.10 (d, J=8.4Hz, 1H, CH); 4.28 (q, 2H, $OCH_2$); 3.60-3.10 (m., 4H, piperidine +$D_2O$); 2.79 (s., 3H, $NCH_3$); 2.60-2.20 (s.b., 2H, piperidine); 2.20-1.80 (s.b., 2H, piperidine); 1.32 (t, 3H, $CH_3$)

**$^{13}$C-NMR ($CDCl_3$, 200 MHz):** 159.24 (C=O); 167.48 (C=O); 133.07 (1C); 132.27 (1C); 128.75 (2C); 127.21 (2C); 65.81 (1C, C4piperidine); 62.29 ($O\underline{C}H_2$); 50.14 and 49.88 (2C, $2NCH_2$); 47.82 (1C); 43.35 ($NCH_3$); 34.30 and 32.91 (2C, $2CH_2$piperidine); 14.14(1C, CH3)

**Example 4. Ethyl (R,S)-2-(4-chlorobenzamido)-2-(4-mercapto-1-methylpiperidin-4-yl)acetate 2,2,2-trifluoroacetate**

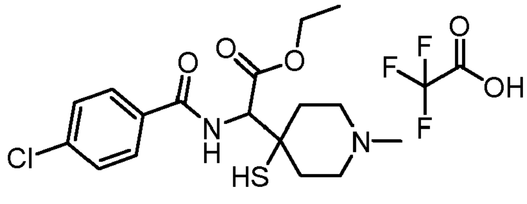

**$^1$H -NMR ($CDCl_3$, 200 MHz):** 7.82 (d, J=6Hz, 2H); 7.40 (d, J=6Hz, 2H); 7.24 (d, J=10Hz, NH); 5.01 (d, J=10Hz, 1H, CH); 4.25 (q, 2H, $OCH_2$); 3.51-3.00 (m, 4H, piperidine); 2.82 (s., $NCH_3$); 2.70-2.40 (m, 2H, piperidine); 2.10-1.80 (m, 2H, piperidine); 1.27 (t, 3H, $CH_3$)

**$^{13}$C-NMR ($CDCl_3$, 200 MHz):** 169.19 (C=O); 166.59 (C=O); 138.38 (1C); 131.49 (1C); 128.84 (2C); 128.78 (2C); 62.21 ($O\underline{C}H_2$); 61.24 (1C, C4piperidine); 50.19 and 49.85 (2C, $2NCH_2$); 47.58 (1C); 43.41 ($NCH_3$); 33.57 and 32.21 (2C, $2CH_2$piperidine); 14.07 (1C, $CH_3$)

**Example 5. Ethyl (R,S)-2-(2-chlorobenzamido)-2-(4-mercapto-1-methylpiperidin-4-yl)acetate 2,2,2-trifluoroacetate**

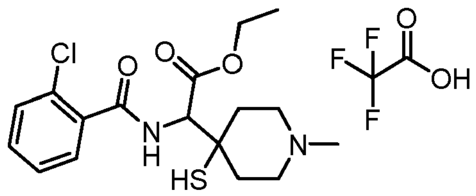

Yield last step: 66%.

**$^1$H -NMR (DMSO-$d_6$, 200 MHz):** 10.20-9.90 (s.b., 1H $CO_2H$); 9.00 (d, NH); 7.50-7.41 (m, 4H); 4.78 (d, 1H, CH); 4.17 (q, 2H, $OCH_2$); 3.60-3.10 (m, 4H, piperidine); 2.81 (s, $NCH_3$); 2.20-1.90 (m, 4H, piperidine); 1.24 (t, 3H, $CH_3$)

**$^{13}$C-NMR (DMSO-$d_6$, 200 MHz):** 168.40 (C=O); 166.88 (C=O); 136.03 (1C); 131.09 (1C); 129.97 (1C); 129.53 (1C); 129.11 (1C); 126.98 (1C); 62.07 (1C, C4piperidine); 61.07 ($O\underline{C}H_2$); 49.35 (2C, $2NCH_2$); 46.21 (1C); 42.45 ($NCH_3$); 33.06 and 32.56 (2C, $2CH_2$piperidine); 14.02 (1C, $CH_3$)

**Example 6. Ethyl (R,S)-2-(4-mercapto-1-methylpiperidin-4-yl)-2-(nicotinamido)** acetate 2,2,2-trifluoroacetate

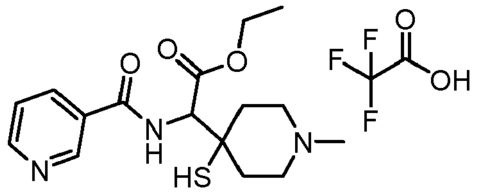

Yield last step: 73%.

**$^1$H -NMR (DMSO-$d_6$, 200 MHz):** 10.20-9.90 (s.b. $CO_2H$); 9.03-8.96 (m., 2H); 8.74 (s.b., 1H); 8.25 (m, 1H); 7.52 (m, 1H); 4.85 (d, 1H, CH); 4.18 (q, 2H, $OCH_2$); 3.50-3.10 (m, 4H, piperidine); 2.81 (s., $NCH_3$); 2.20-1.90 (m, 4H, piperidine); 1.20 (t, 3H, $CH_3$)

**$^{13}$C-NMR (DMSO-$d_6$, $CDCl_3$, 200 MHz):** 168.57 (C=O); 166.13 (C=O); 159.14 and 158.51 ($CO_2H$, TFA); 152.24 (1C); 148.86 (1C); 135.70 (1C); 129.43 (1C); 123.36 (1C); 120.00 and 114.07 (CF3); 62.09 (1C, C4piperidine); 61.13 ($O\underline{C}H_2$); 49.54 and 49.38 (2C, $2NCH_2$); 46.37 (1C, CH); 42.41 ($NCH_3$); 33.02 and 32.21 (2C, $2CH_2$piperidine); 14.01 (1C, $CH_3$)

**Example 7.**

**7a) Ethyl (R,S)-2-(4-mercapto-1-methylpiperidin-4-yl)-2-(phenyl sulfonamido)acetate 2,2,2-trifluoroacetate**

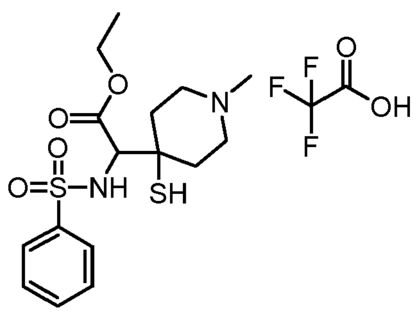

Yield last step: 96%.

**$^1$H -NMR (DMSO-$d_6$, 200 MHz):** 10.20-9.80 (s.b. $CO_2H$); 8.58 (d., J=9.8Hz; NH); 7.80-7.77 (m, 2H); 7.64-7.53 (m, 3H); 3.89 (d., J=10.4Hz; 1H, CH); 3.64 (q, 2H, $OCH_2$); 3.40-3.00 (m, 4H, piperidine); 2.79 (s, $NCH_3$); 2.20-1.60 (m, 4H, piperidine); 0.89 (t, 3H, $CH_3$)

**$^{13}$C-NMR (DMSO-$d_6$, 200 MHz):** 167.88 (C=O); 140.48 (1C); 132.68 (1C); 129.04 (2C); 126.62 (2C); 65.27 (1C, CH); 60.76 (O<u>C</u>$H_2$); 49.43 and 49.29 (2C, $2NCH_2$); 46.29 (1C, C4 of piperidine); 42.47 ($NCH_3$); 32.75 and 31.42 (2C, $2CH_2$piperidine); 13.52 (1C, $CH_3$)

By a process similar to that described in 1f, the corresponding hydrochloride was obtained:

**7b) Ethyl (R,S)-2-(4-mercapto-1-methylpiperidin-4-yl)-2-(phenyl sulfonamido)acetate hydrochloride**

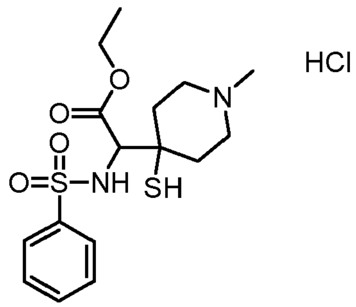

To 9.73g (20mmol) of the product obtained in 7a, 60mL of hydrochloric acid 2N were added. After 5 minutes a white solid appeared and 30 mL of water were added in order to maintain a good stirring. After 15 minutes the solid was filtered and dried to afford 6.3 g of the title product.

Yield last step: 96%. HPLC purity 98.7%

**$^1$H -NMR (DMSO-$d_6$, 200 MHz):** 11.00-10.70 (s.b. HCl); 8.57 (d., J=10.2Hz; NH); 7.80-7.76 (m, 2H); 7.62-7.51 (m, 3H); 3.87 (d., J=10.2Hz; 1H, CH); 3.64 (q, 2H, $OCH_2$); 3.40-3.00 (m, 4H, piperidine); 2.72 (s, $NCH_3$); 2.40-1.60 (m, 4H, piperidine); 0.88 (t, 3H, $CH_3$)

**$^{13}$C-NMR (DMSO-$d_6$, 200 MHz):** 167.82 (C=O); 140.44 (1C); 132.63 (1C); 129.01 (2C); 126.62 (2C); 65.45 (1C, CH); 60.79 (O<u>C</u>$H_2$); 49.15 and 48.98 (2C, $2NCH_2$); 46.57 (1C, C4 of piperidine); 42.25 ($NCH_3$); 32.67 and 32.02 (2C, $2CH_2$piperidine); 13.56 (1C, $CH_3$)

**7c) Ethyl (R,S)-2-(4-mercapto-1-methylpiperidin-4-yl)-2-(phenyl sulfonamido)acetate**

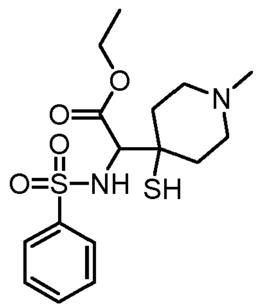

To 19.46g (40mmol) of the product obtained in 7a and 100mL of water, 40mL of 1N sodium hydroxide were added. After 5 minutes the solution was extracted with 3x100mL of $CH_2Cl_2$ and the extracts were dried with sodium sulphate, filtered and concentrated to yield 14.5g of the title product as a white solid.

Yield last step: 97.3%. HPLC purity 94.8%

**$^1$H -NMR (DMSO-$d_6$, 200 MHz):** 7.80-7.78 (m, 2H); 7.63-7.55 (m, 3H); 3.87 (s., 1H, CH); 3.66 (q, 2H, $OCH_2$); 2.60-2.20 (m, 4H, piperidine); 2.14 (s, $NCH_3$); 2.00-1.40 (m, 4H, piperidine); 0.91 (t, 3H, $CH_3$)

**$^{13}$C-NMR ($DMSO-d_6$, 200 MHz):** 168.03 (C=O); 140.67 (1C); 132.49 (1C); 128.93 (2C); 126.57 (2C); 65.48 (1C, CH); 60.44 (O<u>C</u>$H_2$); 50.59 and 50.48 (2C, $2NCH_2$); 48.15 (1C, C4 of piperidine); 45.74 ($NCH_3$); 35.34 and 34.41 (2C, $2CH_2$piperidine); 13.61 (1C, $CH_3$)

**7d) Ethyl 2-(4-mercapto-1-methylpiperidin-4-yl)-2-(phenylsulfonamido) acetate, isomer E2 with L(+) tartaric acid**

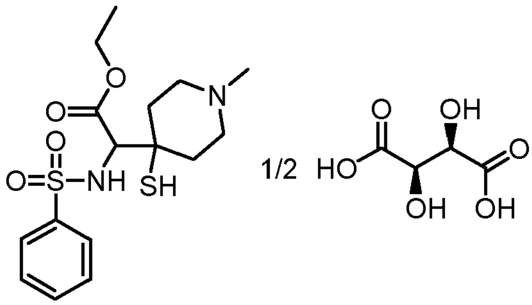

7.45g (20mmol) of the product obtained in 7c and 150mL of ethanol were heated to reflux. Then a solution of 0.75g (5mmol) of L(+) tartaric acid in 50mL of ethanol was added. The solution was cooled slowly with stirring and after 3h it was filtered to afford 4.1g of a white solid (ee=76% by HPLC) and 200 mL of an ethanolic solution. The solid was recrystallized in 150mL of ethanol to yield 3.6g (80%) of the title product with an enantiomeric excess of 93.2%

**$^{1}$H -NMR ($DMSO-d_6$, 200 MHz):** 7.80-7.76 (m, 2H); 7.63-7.52 (m, 3H); 6.20-4.60 (s.b., 4H, OH and $CO_2H$ of tartaric acid and NH and SH from the title product); 4.06 (s, 1H, tartaric acid); 3.90 (s., 1H, CH); 3.72 (q, 2H, $OCH_2$); 3.00-2.50 (m, 4H, piperidine); 2.37 (s, $NCH_3$); 2.20-1.50 (m, 4H, piperidine); 0.91 (t, 3H, $CH_3$)

**$^{13}$C-NMR ($DMSO-d_6$, 200 MHz):** 174.39 (2C, C=O, tartaric); 168.19 (C=O); 140.59 (1C); 132.58 (1C); 128.99 (2C); 126.61 (2C); 71.87 (2C, tartaric); 65.20 (1C, CH); 60.58 (O<u>C</u>$H_2$); 49.98 and 49.83 (2C, $2NCH_2$); 47.46 (1C, C4 of piperidine); 44.28 ($NCH_3$); 34.20 and 33.18 (2C, $2CH_2$piperidine); 13.61 (1C, $CH_3$)

**7e) Ethyl 2-(4-mercapto-1-methylpiperidin-4-yl)-2-(phenylsulfonamido) acetate, isomer E1 with D(-) tartaric acid**

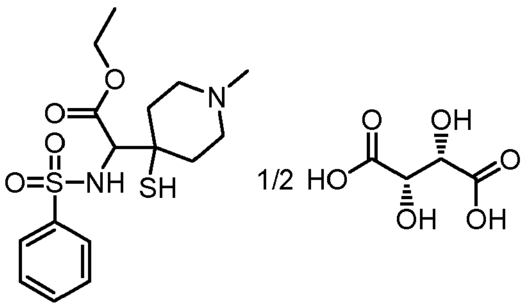

To the 200ml of the ethanolic solution obtained in 7d, 0.67g (4.5mmol) of D(-) tartaric acid were added. The solution was stirred and after 3h it was filtered to afford 4.1g of a white solid (ee=78% by HPLC). The solid was recrystallized in 120mL of ethanol to yield 3.65 g of the title product with an enantiomeric excess of 86.8%

**$^{1}$H -NMR ($DMSO-d_6$, 200 MHz):** 7.85-7.49 (m, 5H); 7.00-4.60 (s.b., 4H, OH and $CO_2H$ of tartaric acid and NH and SH from the title product); 4.08 (m, 1H, tartaric acid); 3.90 (s., 1H, CH); 3.72 (q, 2H, $OCH_2$); 3.00-2.50 (m, 4H, piperidine); 2.42 (s, $NCH_3$); 2.20-1.50 (m, 4H, piperidine); 0.99 (t, 3H, $CH_3$)

**$^{13}$C-NMR ($DMSO-d_6$, 200 MHz):** 175.98 (2C, C=O, tartaric); 167.65 (C=O); 140.57 (1C); 132.58 (1C); 128.98 (2C); 126.62 (2C); 72.13 (2C, tartaric); 65.16 (1C, CH); 60.61 (O<u>C</u>$H_2$); 49.82 and 49.64 (2C, $2NCH_2$); 47.34 (1C, C4 of piperidine); 43.94 ($NCH_3$); 33.95 and 33.01 (2C, $2CH_2$piperidine); 13.61 (1C, $CH_3$)

**Example 8. Ethyl (R,S)-2-(4-mercapto-1-methylpiperidin-4-yl)-2-(4-nitrophenyl sulfonamido)acetate 2,2,2-trifluoroacetate**

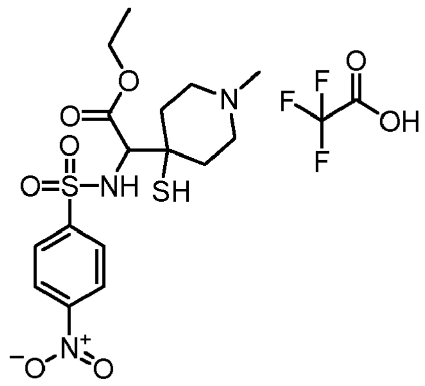

Yield last step: 64%.

**$^1$H -NMR (DMSO-$d_6$, 200 MHz):** 10.00-9.80 (s.b. $CO_2H$); 9.10-8.90 (s.b., NH); 8.41 (d, 2H); 8.05 (d, 2H); 4.00 (s.b., 1H, CH); 3.74 (q, 2H, $OCH_2$); 3.40-3.10 (m, 4H, piperidine); 2.78 (s, $NCH_3$); 2.40-1.70 (m, 4H, piperidine); 0.92 (t, 3H, $CH_3$)

**$^{13}$C-NMR (DMSO-$d_6$, 200 MHz):** 167.73 (C=O); 149.69 (1C); 146.04 (1C); 128.35 (2C); 124.40 (2C); 65.27 (1C, CH); 60.98 ($O\underline{C}H_2$); 49.25 (2C, $2NCH_2$); 46.25 (1C, C4 of piperidine); 42.47 ($NCH_3$); 32.75 and 31.50 (2C, $2CH_2$piperidine); 13.52 (1C, $CH_3$)

**Example 9. Ethyl (R,S)-2-(4-mercapto-1-methylpiperidin-4-yl)-2-(4-methoxyphenyl sulfonamido)acetate 2,2,2-trifluoroacetate**

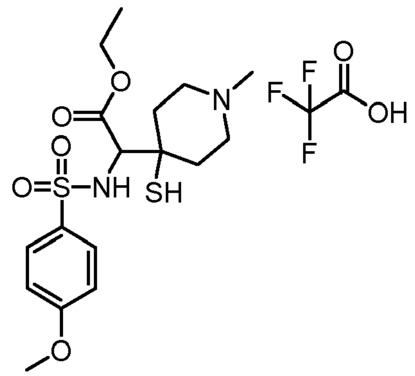

**$^1$H -NMR (DMSO-$d_6$, 200 MHz):** 9.90-9.70 (s.b. $CO_2H$); 8.40 (d., J=10.2Hz, NH); 7.71 (d, 2H); 7.09 (d, 2H); 3.82 (s, 3h, $OCH_3$); 3.79 (d., 1H, CH); 3.64 (q, 2H, $OCH_2$); 3.50-3.10 (m, 4H, piperidine); 2.79 (s, $NCH_3$); 2.20-1.70 (m, 4H, piperidine); 0.93 (t, 3H, $CH_3$)

**$^{13}$C-NMR (DMSO-$d_6$, 200 MHz):** 168.05 (C=O); 162.39 (1C); 132.13 (1C); 128.86 (2C); 114.12 (2C); 65.17 (1C, CH); 60.80 ($O\underline{C}H_2$); 55.70 (1C, $OCH_3$); 49.46 and 49.29 (2C, $2NCH_2$); 46.24 (1C, C4 of piperidine); 42.47 ($NCH_3$); 32.75 and 31.32 (2C, $2CH_2$piperidine); 13.50 (1C, $CH_3$)

**Example 10. Ethyl (R,S)-2-(4-mercapto-1-methylpiperidin-4-yl)-2-(4-methylphenyl sulfonamido)acetate 2,2,2-trifluoroacetate**

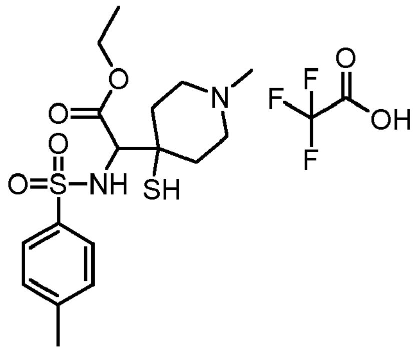

**$^1$H -NMR (DMSO-$d_6$, 200 MHz):** 9.90-9.70 (s.b. $CO_2H$); 8.48 (d., J=10.2Hz, NH); 7.66 (d, 2H); 7.37 (d, 2H); 3.88 (d., 1H, J=10.2Hz, CH); 3.67 (q, 2H, $OCH_2$); 3.50-3.10 (m, 4H, piperidine); 2.79 (s, $NCH_3$); 2.37 (s, 3H, $CH_3$); 2.20-1.70 (m, 4H, piperidine); 0.94 (t, 3H, $CH_3$)

**$^{13}$C-NMR (DMSO-$d_6$, 200 MHz):** 167.97 (C=O); 143.00 (1C); 137.62 (1C); 129.40 (2C); 126.67 (2C); 65.19 (1C, CH); 60.75 ($O\underline{C}H_2$); 49.46 and 49.30 (2C, $2NCH_2$); 46.23 (1C, C4 of piperidine); 42.47 ($NCH_3$); 32.77 and 31.25 (2C, $2CH_2$piperidine); 20.93 (1C, $CH_3$); 13.43 (1C, $CH_3$)

**Example 11. Ethyl (R,S)-2-(2-chlorophenylsulfonamido)-2-(4-mercapto-1-methylpiperidin-4-yl)acetate 2,2,2-trifluoroacetate**

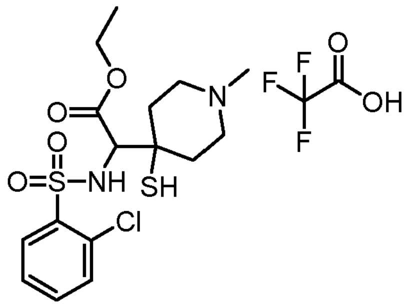

**$^1$H -NMR (DMSO-$d_6$, 200 MHz):** 9.80-9.50 (s.b. $CO_2H$); 8.62 (d., J=10Hz, NH); 7.99 (d, 1H); 7.67-7.50 (m, 3H); 3.96 (d., 1H, J=10.2Hz, CH); 3.79 (q, 2H, $OCH_2$); 3.50-3.10 (m, 4H, piperidine); 2.80 (s, $NCH_3$); 2.30-1.83 (m, 4H, piperidine); 0.96 (t, 3H, $CH_3$)

**$^{13}$C-NMR (DMSO-$d_6$,** 200 MHz): 167.80 (C=O); 137.43 (1C); 134.42 (1C); 131.71 (1C); 130.93 (1C); 130.87 (1C); 127.65 (1C); 65.54 (1C, CH); 60.98 ($O\underline{C}H_2$); 49.52 and 49.36 (2C, $2NCH_2$); 46.27 (1C, C4 of piperidine); 42.50 ($NCH_3$); 32.95 and 31.68 (2C, $2CH_2$ piperidine); 13.58 (1C, $CH_3$)

**Example 12. Ethyl (R,S)-2-(3-chlorophenylsulfonamido)-2-(4-mercapto-1-methyl piperidin-4-yl)acetate 2,2,2-trifluoroacetate**

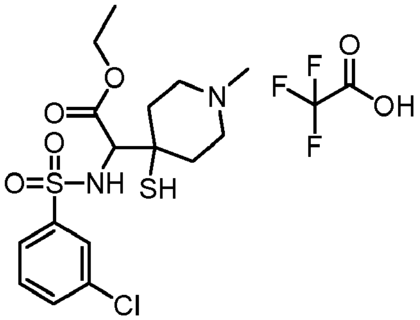

Yield last step: 62%.

**$^1$H -NMR (DMSO-$d_6$, 200 MHz):** 9.90-9.70 (s.b. $CO_2H$); 8.78 (d., J=10Hz, NH); 7.80-7.50 (m, 4H); 3.96 (d., 1H, J=10.2Hz, CH); 3.72 (q, 2H, $OCH_2$); 3.50-3.10 (m, 4H, piperidine); 2.78 (s, $NCH_3$); 2.30-1.60 (m, 4H, piperidine); 0.91 (t, 3H, $CH_3$)

**$^{13}$C-NMR (DMSO-$d_6$, 200 MHz):** 167.80 (C=O); 142.37 (1C); 133.69 (1C); 132.62 (1C); 131.15 (1C); 126.33 (1C); 125.30 (1C); 65.21 (1C, CH); 60.89 ($O\underline{C}H_2$); 49.42 (2C, $2NCH_2$); 46.23 (1C, C4 of piperidine); 42.47 ($NCH_3$); 32.68 and 31.28 (2C, $2CH_2$, piperidine); 13.49 (1C, $CH_3$)

**Example 13. Ethyl (R,S)-2-(4-chlorophenylsulfonamido)-2-(4-mercapto-1-methyl piperidin-4-yl)acetate 2,2,2-trifluoroacetate**

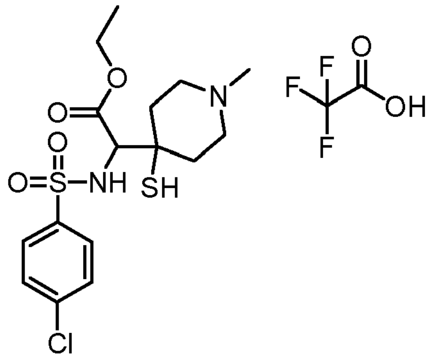

**$^1$H -NMR (DMSO-$d_6$, 200 MHz):** 9.90-9.70 (s.b. $CO_2H$); 8.70 (d., J=10Hz, NH); 7.82 (d, 2H); 7.67 (d, 2H); 3.96 (d, 1H, J=10.2Hz, CH); 3.72 (q, 2H, $OCH_2$); 3.50-3.10 (m, 4H, piperidine); 2.79 (s, $NCH_3$); 2.30-1.70 (m, 4H, piperidine); 0.92 (t, 3H, $CH_3$)

**$^{13}$C-NMR (DMSO-$d_6$, 200 MHz):** 167.85 (C=O); 139.38 (1C); 137.61 (1C); 129.17 (2C); 128.63 (2C); 65.23 (1C, CH); 60.87 ($O\underline{C}H_2$); 49.41 (2C, $2NCH_2$); 46.24 (1C, C4 of piperidine); 42.46 ($NCH_3$); 32.73 and 31.40 (2C, $2CH_2$, piperidine); 13.47 (1C, $CH_3$)

**Example 14. Ethyl (R,S)-2-(2-fluorophenylsulfonamido)-2-(4-mercapto-1-methyl piperidin-4-yl)acetate 2,2,2-trifluoroacetate**

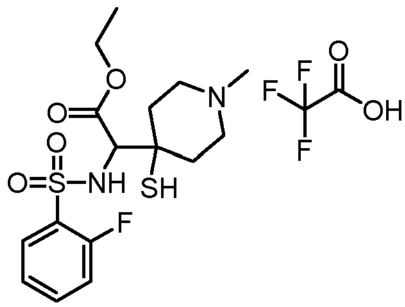

Yield last step: 57%.

**$^1$H -NMR (DMSO-$d_6$,** 200 MHz): 10.10-9.80 (s.b. $CO_2H$); 8.78 (d., NH); 7.82-7.60 (m, 2H); 7.42-7.34 (m, 2H); 3.96 (d, 1H, CH); 3.82 (q, 2H, $OCH_2$); 3.50-3.10 (m, 4H, piperidine); 2.78 (s, $NCH_3$); 2.30-1.80 (m, 4H, piperidine); 0.99 (t, 3H, $CH_3$)

**$^{13}$C-NMR (DMSO-$d_6$, 200 MHz):** 167.80 (C=O); 160.74 and 155.69 (1C, C-F); 135.76 and 135.59 (1C); 129.87 (1C); 128.25 and 127.97 (1C); 124.86 and 124.79 (1C); 117.31 and 116.89 (1C); 65.69 (1C, CH); 61.04 ($O\underline{C}H_2$); 49.49 and 49.31 (2C, $2NCH_2$); 46.35 (1C, C4 of piperidine); 42.43 ($NCH_3$); 32.95 and 32.24 (2C, $2CH_2$piperidine); 13.58 (1C, $CH_3$)

**Example 15. Ethyl (R,S)-2-(4-fluorophenylsulfonamido)-2-(4-mercapto-1-methyl piperidin-4-yl)acetate 2,2,2-trifluoroacetate**

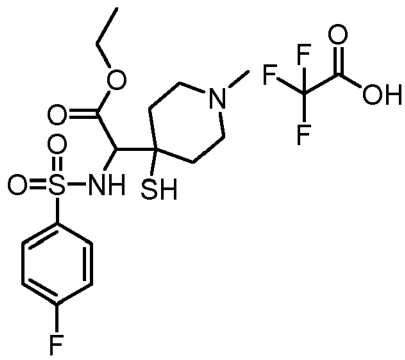

Yield last step: 81%.

**$^1$H -NMR (DMSO-$d_6$, 200 MHz):** 10.10-9.80 (s.b. $CO_2H$) 8.60 (d., NH); 7.89-7.80 (m, 2H); 7.47-7.30 (m, 2H); 3.96 (d, 1H, CH); 3.74 (q, 2H, $OCH_2$); 3.50-3.00 (m, 4H, piperidine); 2.79 (s, $NCH_3$); 2.30-1.70 (m, 4H, piperidine); 0.93 (t, 3H, $CH_3$)

**$^{13}$C-NMR (DMSO-$d_6$, 200 MHz):** 167.90 (C=O); 166.81 and 161.82 (1C, C-F); 158.87 and 158.25 ($CO_2H$) 136.96 and 136.90 (1C, C-$SO_2$-) 129.87 and 129.68 (2C); 116.41 and 115.96 (2C); 120.14 and 114.20 (CF3); 65.23 (1C, CH); 60.89 ($O\underline{C}H_2$); 49.27 (2C, $2NCH_2$); 46.29 (1C, C4 of piperidine); 42.40 ($NCH_3$); 32.68 and 31.50 (2C, $2CH_2$, piperidine); 13.55 (1C, $CH_3$)

**Example 16. Ethyl (R,S)-2-(2,4-difluorophenylsulfonamido)-2-(4-mercapto-1-methyl piperidin-4-yl)acetate 2,2,2-trifluoroacetate**

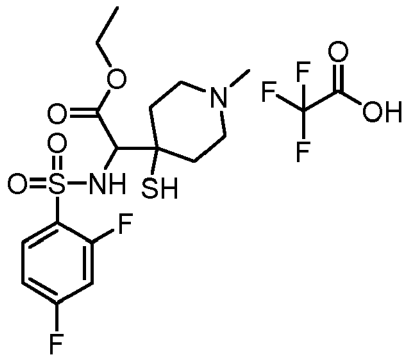

Yield last step: 77%.

**$^1$H -NMR (DMSO-$d_6$, 200 MHz):** 10.20-9.80 (s.b. $CO_2H$); 8.90 (s.b.., NH); 7.90-7.80 (m, 1H); 7.60-7.50 (m, 1H); 7.35-7.25 (m, 1H); 3.96 (s.b.,1H, CH); 3.88 (q, 2H, $OCH_2$); 3.50-3.00 (m, 4H, piperidine); 2.78 (s, $NCH_3$); 2.30-1.80 (m, 4H, piperidine); 0.99 (t, 3H, $CH_3$)

**$^{13}$C-NMR (DMSO-$d_6$, 200 MHz):** 167.81 (C=O); 167.81, 167.60, 161,69 and 161,42 (1C, C-F); 162.73, 162.48, 156.58 and 156.32 (1C, C-F); 158.65 and 158.03 ($CO_2H$) 132.09 and 131.88 (1C); 125.11, 125.03, 124.83 and 124.74 (1C); 112.38, 112.32, 111.94 and 111.88 (1C); 120.13 and 114.17 (CF3); 106.33, 105.81 and 105.29 (1C); 65.58 (1C, CH); 61.10 ($O\underline{C}H_2$); 49.48 and 49.30 (2C, $2NCH_2$); 46.26 (1C, C4 of piperidine); 42.44 ($NCH_3$); 32.91 and 32.10 (2C, $2CH_2$, piperidine); 13.59 (1C, $CH_3$)

**Example 17.**

**Synthesis of Ethyl (R,S)-2-formamido-2-(4-mercapto-1-methylpiperidin-4-yl)acetate 2,2,2-trifluoroacetate**

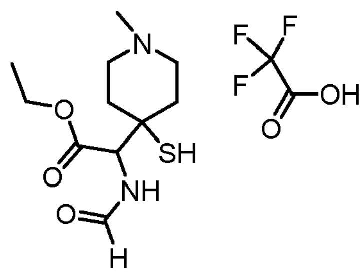

3.8 g (10 mmol) of the product obtained in 1b and 10 ml of trifluoroacetic acid were added to a 50 ml flask and heated under reflux for 20 hours. The mixture was allowed to cool and 20 ml of water were added and washed with 3x50 ml of AcOEt. The aqueous phase was concentrated to dryness by azeotropically distilling the remains of water with IPA. The residue was treated with $Et_2O$ and stirring to afford a white solid, which is filtered and dried. 3.6 g of the title product were obtained (Yield: 83%)

**$^1$H -NMR (DMSO-$d_6$, 200 MHz):** 9.90-9.70 (s.b., 1H, $CO_2H$) 8.80 (d, 1H, NH); 4.60 (d, 1H, CH); 4.14 (q, 2H, $OCH_2$); 3.50-3.10 (m, 4H, piperidine); 2.79 (s, 3H, $NCH_3$); 2.20-1.60 (m, 4H, piperidine); 1.19 (t, 3H, $CH_3$)

**$^{13}$C-NMR (DMSO-$d_6$, 200 MHz):** 168.45 (C=O); 161.44 (C=O); 158.99 and 158.27 ($CO_2H$) 118.84 and 113.03 (CF3); 62.07 (1C, CH); 61.19 ($O\underline{C}H_2$); 49.53 and 49.38 (2C, 2 $NCH_2$); 46.46 (1C, C4piperidine); 42.44 ($NCH_3$); 33.12 and 32.50 (2C, $2CH_2$); 13.98 (1C, $CH_3$)

**Example 18.**

**Synthesis of Ethyl (R,S)-2-(4-mercapto-1-methylpiperidin-4-yl)-2-(2,2,2-trifluoroacetamido)acetate 2,2,2-trifluoroacetate(INT8030TFA)**

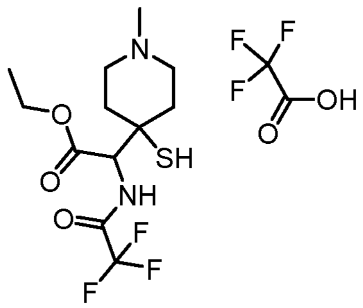

The product of the title is obtained starting from the product obtained in Example 1c and proceeding as described in Example 1e or 17.

Yield: 30%.

**$^1$H -NMR (DMSO-$d_6$, 200 MHz):** 9.91 (d, J=8Hz, 1H, NH); 4.67 (d, J=8Hz, 1H, CH); 4.16 (q, 2H, $OCH_2$); 3.50-3.10 (m, 4H, piperidine); 2.79 (s, 3H, $NCH_3$); 2.20-1.80 (m, 4H, piperidine); 1.20 (t, 3H, $CH_3$)

**$^{13}$C-NMR (DMSO-$d_6$, 200 MHz):** 167.29 (C=O); 118.84 (CF3); 62.34 (1C, CH); 61.49 (O<u>C</u>$H_2$); 49.23 (2C, 2 $NCH_2$); 45.96 (1C, C4piperidine); 42.39 ($NCH_3$); 32.75 and 32.18 (2C, 2$CH_2$); 13.87 (1C, $CH_3$)

MS: e/m = 329.4 (M+1) from the free base

**Example 19.**

**19a) Synthesis of Benzyl (R,S)-amino-[1-methyl-4-(4-methoxy-benzylsulfanyl)-piperidin-4-yl]-acetate**

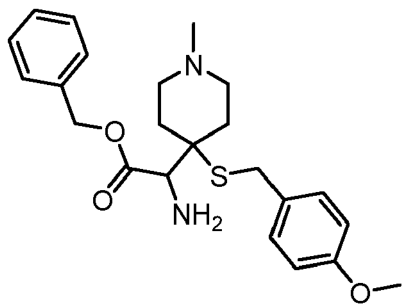

The product of the title is obtained starting from the product obtained in Example 1c by treatment with benzyl alcohol at 100°C.

**$^1$H -NMR (DMSO-$d_6$, 200 MHz):** 7.41-7.33 (m,5H); 7.10 (d, J=8.8Hz, 2H); 6.79 (d, J=8.8Hz, 2H); 5.14 (s, 2H, $OCH_2Ph$); 3.70 (s, 3H, $OCH_3$); 3.60-3.40 (m, CH + $SCH_2$); 2.50 (s.b., 2H, $NH_2$); 2.40-2.20 (m, 2H, piperidine); 2.15 (s, 3H, $NCH_3$); 2.10-1.50 (m, 6H, piperidine).

**$^{13}$C-NMR (DMSO-$d_6$, 200 MHz):** 173.34 (C=O); 158.13 (C-O); 135.90 (1C, C-$CH_2$O); 130.15 (2C); 129.28 (1C C-$CH_2$S); 128.41-128.07 (5C); 113.73 (2C, CH); 65.67 (O<u>C</u>$H_2$); 61.93 (1C, CH); 54.99 (1C, $OCH_3$); 51.20 and 50.58 (3C, 2$NCH_2$ and C4piperidine); 45.86 ($NCH_3$); 30.29 and 30.15 (3C, 2$CH_2$ and S-$CH_2$)

**19b) Synthesis of Benzyl (R,S)-2-acetamido-2-(4-mercapto-1-methylpiperidin-4-yl)acetate 2,2,2-trifluoroacetate**

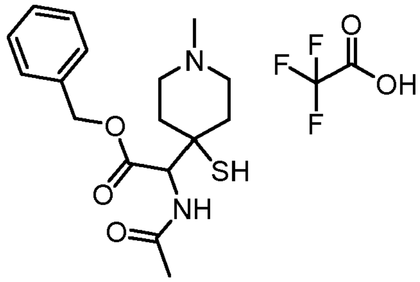

The product of the title is obtained starting from the product obtained in 19a and following the processes described in 1d and 1e.

**$^1$H -NMR ($CDCl_3$, 200 MHz):** 7.27 (s, 5H); 5.11 (s, 2H, $OCH_2$); 4.74 (s, 1H, CH); 3.50-3.10 (m, 4H, piperidine); 2.76 (s, $NCH_3$); 2.30-1.80 (m, 4H, piperidine); 1.96 (s, 3H, $COCH_3$);

**$^{13}$C-NMR ($CDCl_3$, 200 MHz):** 171.61 (CO); 168.34 (C=O); 134.47 (1C); 128.04 and 127.96 (5C); 66.96 (O$\underline{C}H_2$); 61.01 (C4piperidine); 49.82 and 49.54 (2C, $2NCH_2$); 45.85 (1C); 42.47 ($NCH_3$); 33.09 and 31.87 (2C, $2CH_2$piperidine); 21.36 (1C, $COCH_3$)

The following products were obtained by following a process similar to that described in example 1 but substituting 1-methyl-4-piperidone with 1-ethyl-4-piperidone or 1-benzyl-4-piperidone in step a):

**Example 20. Synthesis of Ethyl (R,S)-2-acetamido-2-(1-ethyl-4-mercaptopiperidin-4-yl)acetate 2,2,2-trifluoroacetate**

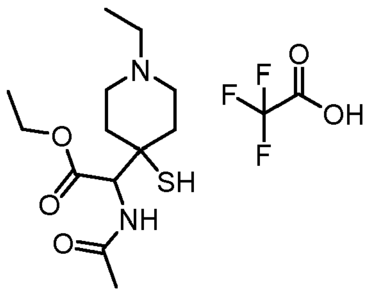

Yield last step: 26%

**$^1$H -NMR (DMSO-$d_6$, 200 MHz):** 9.80-9.20 (s.b., 1H, $CO_2H$); 8.41 (d, J=9.2Hz, 1H, NH); 4.57 (d, J=9.2Hz, 1H, CH); 4.14 (m, 4H, $OCH_2$ + $NCH_2$); 3.20-3.00 (m, 4H, piperidine); 1.90 (s, 3H, $COCH_3$); 2.20-1.80 (m, 4H, piperidine); 1.22 (t, 3H, $CH_3$); 1.02 (t, 3H, $CH_3$)

**$^{13}$C-NMR (DMSO-$d_6$, 200 MHz):** 169.87 (C=O); 168.95 (C=O); 158.99 and 158.25 ($CO_2H$) 118.3 and 112.60 (CF3); 61.42 (1C, CH); 60.92 (O$\underline{C}H_2$); 50.90 (1C, C4 piperidine); 47.36 and 46.90 (2C, $2NCH_2$); 47.11 ($NCH_2$); 32.98 and 32.09 (2C, $2CH_2$); 22.25 (1C, $COCH_3$); 13.99 (1C, OCH2$\underline{CH_3}$); 8.99 (1C, $NCH_2\underline{CH_3}$)

**Example 21. Synthesis of Ethyl (R,S)-2-(1-ethyl-4-mercaptopiperidin-4-yl)-2-(nicotinamido)acetate 2,2,2-trifluoroacetate**

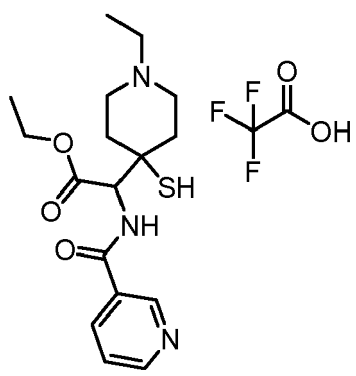

Yield last step: 19%

**$^1$H -NMR (DMSO-$d_6$, 200 MHz):** 9.70-9.50 (s.b. $CO_2H$); 9.03-8.96 (m., 2H); 8.74 (d., 1H); 8.22 (d, 1H); 7.52 (m, 1H); 4.85 (d, 1H, CH); 4.18 (q, 2H, $OCH_2$); 3.50-3.10 (m, 6H, 4Hpiperidine + $CH_2N$); 2.20-1.90 (m, 4H, piperidine); 1.20 (t, 3H, $CH_3$); 1.06 (t, 3H, $CH_3$)

**$^{13}$C-NMR (DMSO-$d_6$, $CDCl_3$, 200 MHz):** 168.58 (C=O); 166.07 (C=O); 152.27 (1C); 148.84 (1C); 135.68 (1C); 129.40 (1C); 123.39 (1C); 62.10 (1C, CH); 61.12 ($O\underline{C}H_2$); 50.89 (1C, C4piperidine); 47.42 and 47.12 (2C, $2NCH_2$); 46.88 ($NCH_2$); 33.00 and 32.13 (2C, $2CH_2$piperidine); 14.01 (1C, $CH_3$); 9.02 (1C, $CH_3$)

**Example 22. Synthesis of Ethyl (R,S)-2-acetamido-2-(1-benzyl-4-mercaptopiperidin-4-yl)acetate 2,2,2-trifluoroacetate**

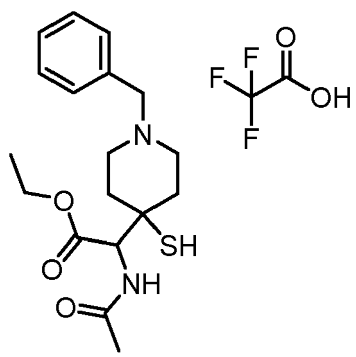

Yield last step: 52%

**$^1$H -NMR (DMSO-$d_6$, 200 MHz):** 10.20-9.90 (s.b. $CO_2H$); 8.40 (d, J=9.2Hz, NH); 7.51-7.42 (m, 5H, phenyl); 4.56 (d, J=9.2Hz, 1H, CH); 4.33 (b.s., 2H, $NCH_2Ph$); 4.08 (q, 2H, $OCH_2$); 3.40-3.00 (m, 4H, piperidine); 2.20-1.70 (m, 4H, piperidine); 1.89 (s, 3H, $COCH_3$); 1.14 (t, 3H, $CH_3$)

**$^{13}$C-NMR (DMSO-$d_6$, 200 MHz):** 169.84 (C=O); 168.96 (C=O); 131.30, 129.58 and 128.85 (6C, Phenyl); 61.32 (1C, CH); 60.92 ($O\underline{C}H_2$); 58.91(1C, C4piperidine); 47.59 and 47.29 (2C, $2NCH_2$); 46.90 (1C, $NCH_2Ph$); 32.81 and 31.84 (2C, $2CH_2$); 22.27 (1C, $COCH_3$); 13.98 (1C, $CH_3$)

**Example 23. Synthesis of Ethyl (R,S)-2-(1-benzyl-4-mercaptopiperidin-4-yl)-2-(nicotinamido)acetate 2,2,2-trifluoroacetate**

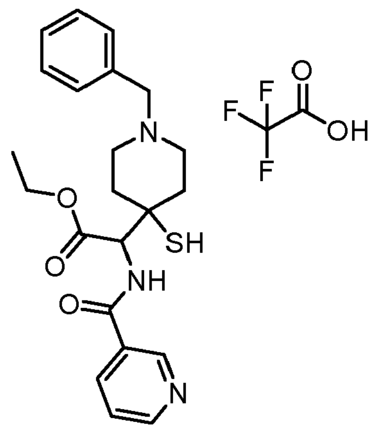

**$^1$H -NMR (DMSO-$d_6$, 200 MHz):** 10.10-9.90 (s.b., $CO_2H$); 9.10-8.90 (m, 2H); 8.72 (d, 1H); 8.21 (d, J=8Hz, 1H); 7.60-7.42 (m, 6H, 1Hpyridine +5HPh); 4.85 (d, J=9.2Hz, 1H, CH); 4.36 (s.b., 2H, $CH_2$Ph); 4.09 (q, 2H, $OCH_2$); 3.40-3.00 (m, 4H, piperidine); 2.20-1.90 (m, 4H, piperidine); 1.19 (t, 3H, $CH_3$)

**$^{13}$C-NMR (DMSO-$d_6$, 200 MHz):** 168.52 (C=O); 165.98 (C=O); 152.10 (1C, pyridine); 148.70 (2C,pyridine); 135.77 (1C,pyridine); 131.33, 129.58 and 128.82 (6C, Phenyl); 123.45 (1C,pyridine); 62.05 (1C, CH); 61.10 ($O\underline{C}H_2$); 58.89 (1C, C4piperidine); 47.59 and 47.33 (2C, $2NCH_2$); 46.86 (1C, $NCH_2$); 32.84 and 31.98 (2C, $2CH_2$piperidine); 13.96 (1C, $CH_3$).

The following products were obtained by a process similar to that described in example 17 but starting from the corresponding N-ethyl or N-benzylpiperidine compound:

**Example 24. Synthesis of Ethyl (R,S)-2-(1-ethyl-4-mercaptopiperidin-4-yl)-2-formamidoacetate 2,2,2-trifluoroacetate**

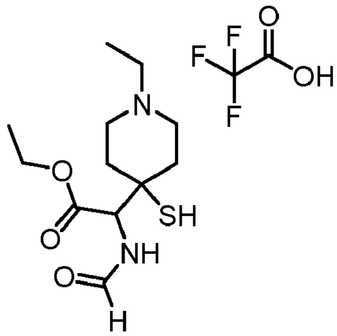

Yield last step: 60%

**$^1$H -NMR (DMSO-$d_6$, 200 MHz):** 9.80-9.60 (s.b., $CO_2H$); 8.78 (d, J=9.6Hz, NH); 8.12 (s, 1H, CHO); 4.60 (d, J=9.4Hz, 1H, CH); 4.09 (q, 2H, $OCH_2$); 3.50-3.00 (m, 6H, 4Hpiperidine +$NCH_2$); 2.20-1.80 (m, 4H, piperidine); 1.06 (t, 3H, $CH_3$); 0.97 (t, 3H, $CH_3$)

**$^{13}$C-NMR (DMSO-$d_6$, 200 MHz):** 168.49 (C=O); 161.42 (C=O); 61.14 (1C, CH); 59.90 ($O\underline{C}H_2$); 50.89 (1C, C4piperidine); 47.31 and 47.09 (2C, $2NCH_2$); 46.92 (1C, $NCH_2$); 32.98 and 32.34 (2C, $2CH_2$); 13.97 (1C, $CH_3$); 9.01 (1C, $CH_3$);

**Example 25. Synthesis of Ethyl (R,S)-2-(1-benzyl-4-mercaptopiperidin-4-yl)-2-formamidoacetate 2,2,2-trifluoroacetate**

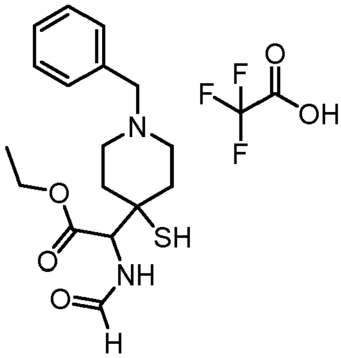

Yield last step: 45%

**$^1$H -NMR (DMSO-$d_6$, 200 MHz):** 9.90-9.70 (s.b., $CO_2H$); 8.78 (d, NH); 8.11 (s, 1H, CHO); 7.46 (s.b, 5H, phenyl); 4.50 (d, 1H, CH); 4.34 (s.b., 2H, $NCH_2Ph$); 4.11 (q, 2H, $OCH_2$); 3.50-3.00 (m, 4H, piperidine); 2.20-1.80 (m, 4H, piperidine); 1.19 (t, 3H, $CH_3$)

**$^{13}$C-NMR (DMSO-$d_6$, 200 MHz):** 168.44 (C=O); 161.40 (C=O); 131.32, 129.60 and 128.85 (6C, Phenyl); 61.13 (O<u>C</u>$H_2$); 59.81 (1C, CH); 58.91 (1C, C4piperidine); 47.56 and 47.30 (2C, $2NCH_2$); 46.88 (1C, $NCH_2Ph$); 32.81 and 32.01 (2C, $2CH_2$); 13.95 (1C, $CH_3$)

The following products were obtained by following a process similar to that described in example 1 but substituting ethyl isocyanoacetate with methyl isocyanoacetate in step a):

**Example 26. Synthesis of Methyl (R,S)-2-acetamido-2-(4-mercapto-1-methylpiperidin -4-yl)acetate 2,2,2-trifluoroacetate**

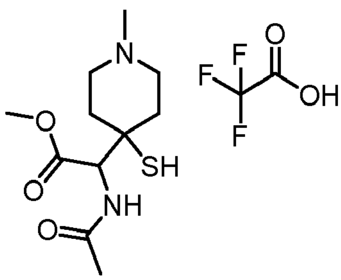

**$^1$H -NMR (DMSO-$d_6$, 200 MHz):** 9.90-9.70 (s.b., $CO_2H$); 8.43 (d, J=9Hz, NH); 4.62 (d, J=9Hz, 1H, CH); 3.66 (s, 3H, $OCH_3$); 3.50-3.10 (m, 4H,piperidine); 2.79 (s, 3H, $NCH_3$); 1.92 (s, 3H, $COCH_3$); 2.20-1.80 (m, 4Hpiperidine);

**$^{13}$C-NMR (DMSO-$d_6$, 200 MHz):** 169.80 (1C, C=O); 169.42 (1C, C=O); 61.14 (1C, CH); 52.00 (O<u>C</u>$H_3$); 49.51 and 49.35 (2C, $2NCH_2$); 46.31(1C, C4piperidine); 42.37 ($NCH_3$); 33.02 and 32.10 (2C, $2CH_2$); 22.24 (1C, $COCH_3$)

**Example 27. Synthesis of Methyl (R,S)-2-(4-mercapto-1-methylpiperidin-4-yl)-2-(nicotinamido)acetate 2,2,2-trifluoroacetate**

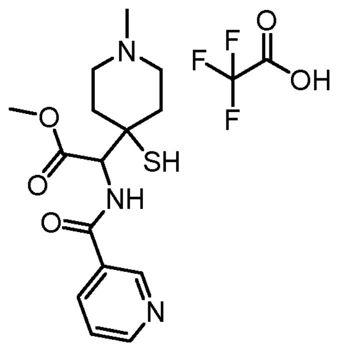

**$^1$H -NMR (DMSO-$d_6$, 200 MHz):** 9.03-8.96 (m. 2H); 8.73 (d, 1Hpyridine); 8.26-8.21 (m, 1H, pyridine); 7.54-7.48 (m, 1H, pyridine); 4.93 (d, J=8.8Hz, 1H, CH); 3.69 (s, 3H, $OCH_3$); 3.50-3.00 (m, 4H, piperidine); 2.79 (s, 3H, $NCH_3$); 2.20-1.80 (m, 4H, piperidine)

**$^{13}$C-NMR (DMSO-$d_6$, 200 MHz):** 168.15 (C=O); 166.13 (C=O); 158.84 and 158.21 ($CO_2H$); 152.31 (1C); 148.92 (1C); 135.69 (1C); 129.36 (1C); 123.36 (1C); 120.18 and 114.60 (CF3); 61.38 (1C, CH); 52.21 (O<u>C</u>$H_3$); 49.50 and 49.28 (2C, $2NCH_2$); 46.30 (1C, C4piperidine); 42.21 (1C, $NCH_3$); 32.87 and 32.18 (2C, $2CH_2$piperidine)

**Example 28. Methyl (R,S)-2-(4-mercapto-1-methylpiperidin-4-yl)-2-(phenylsulfonamido)acetate 2,2,2-trifluoroacetate**

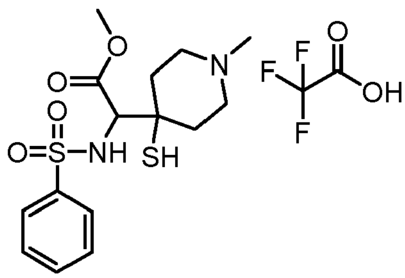

**$^1$H -NMR ($CDCl_3$, 200 MHz):** 10.10-9.90 (s.b., $CO_2H$); 8.57 (d, J=10Hz, NH); 7.80-7.54 (m, 5H); 3.92 (s, J=10Hz, 1H, CH); 3.37 (s, 3H, $OCH_3$); 3.50-3.00 (m, 4H, piperidine); 2.78 (s, $NCH_3$); 2.30-1.70 (m, 4H, piperidine);

**$^{13}$C-NMR($CDCl_3$, 200 MHz):** 168.36 (C=O); 140.26 (1C); 132.68 (1C); 129.01 (2C); 126.61 (2C); 65.35 (1C, CH); 51.64 (1C, $OCH_3$); 49.42 (2C, $2NCH_2$); 46.14 (1C, C4piperidine); 42.44 ($NCH_3$); 32.69 and 31.37 (2C, $2CH_2$piperidine)

The following product were obtained by a process similar to that described in example 17 but starting from the corresponding compound with methyl ester instead of ethyl ester:

**Example 29. Synthesis of Methyl (R,S)-2-formamido-2-(4-mercapto-1-methyl piperidin-4-yl)acetate 2,2,2-trifluoroacetate**

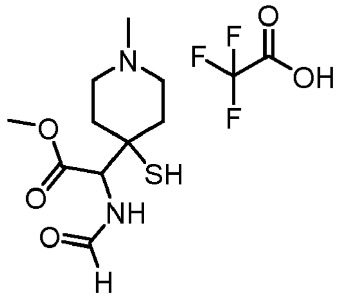

**$^1$H -NMR (DMSO-$d_6$, 200 MHz):** 10.10-9.90 (s.b., $CO_2H$); 8.81 (d, J=9.2Hz, NH); 8.13 (s, 1H, CHO); 4.63 (d, J=9.6Hz, 1H, CH); 3.70 (s, 3H, $OCH_3$); 3.50-3.00 (m, 4H, piperidine); 2.79 (s, $NCH_3$); 2.20-1.80 (m, 4H, piperidine).

**$^{13}$C-NMR (DMSO-$d_6$, 200 MHz):** 168.95 (C=O); 161.42 (C=O); 62.02 (1C, CH); 52.18 (1C, $OCH_3$); 49.46 and 49.30 (2C, $2NCH_2$); 46.36 (1C, C4piperidine); 42.39 ($NCH_3$); 33.01 and 32.43 (2C, $2CH_2$piperidine)

**B) PHARMACOLOGY**

**Material and methods.**

*Ethanol induced gastric lesions in rat*

Ulceration was induced as described by Robert A. (1979) "Cytoprotection by prostaglandins" Gastroenterology, vol. 77, no. 4, part 1, pp. 761—767.

Male Wistar rats (150—200 g) were housed in a standard bioclean animal room, and kept under a 12-hour light-dark cycle at 21—23°C and humidity 50-60%.

The animals were deprived of food for 18 hours before the experiments but free access to water.

Products of the invention were administered intragastrically in the form of a 0.2 mg/ml solution of the product in a vehicle aqueous composition consisting of 1% Tween 80 and 0.5% hydroxypropylmethycellulose in water.

Animals were administered a vehicle solution mentioned above or the vehicle solution comprising the compounds to be tested (compounds of the invention, GSH or GSNO) as a single dose. 30 minutes after the administration, gastric mucosal injury was induced through oral administration of absolute ethanol 4 ml/kg, p.o. After one hour, the animals were sacrificed, stomach was removed, opened along the greater curvature, lightly rinsed with saline, and gastric lesions examined under an illuminated stereomicroscope whereby the maximum length of each ulcer was measured. Animals receiving thiol compounds of the invention or other thiols and having gastric mucosal injury induced by oral administration of absolute ethanol 4 ml/kg, p.o. represented the "treatment group". Animals receiving vehicle instead of thiol and having gastric mucosal injury induced by oral administration of absolute ethanol 4 ml/kg, p.o., following the same procedure as explained for the thiol treated animals, represented the "ethanol group". Animals receiving only vehicle and without induction of gastric mucosal injury by ethanol represented the "sham group". Each group, i.e. sham group, ethanol group and treatment group, consisted of 7 animals (n=7).

Gastric lesions were scored in mm length. The "ulcer score" for a given animal was calculated as the sum of the length of all ulcers (in mm) in said animal.

% Inhibition of ulcers vs ethanol group was calculated for each treatment with the following formula:

% Inhibition vs ethanol group = 100 x (ulcers in ethanol group - ulcers in treated group) / ulcers in ethanol group,

wherein the terms "ulcers in ethanol group" and "ulcers in treated group" refer to the average of the "ulcer score" (in mm) across each group of animals.

The results obtained for the compounds of the invention and reference compounds glutathione (GSH) and S-nitrosoglutathione (GSNO) are shown in Table 1 below.

*Determination of Non-Protein Sulphydryl (NP-SH) Groups*

In the above mentioned experiments, gastric mucus NP-SH concentration was measured according to the method of Sedlak J and Lindsay RH, wherein the estimation of total, protein bound and non-protein sulfhydryl groups

in tissue is determined with Ellman's reagent [Sedlak J and Lindsay RH, Anal Biochem 25:192-5 (1968)], for the treatment group, ethanol group and sham group defined above.

Briefly, the mucus layer of the stomach was weighed and homogenized in icecold 0.02 MEDTA to give 5% homogenates. Aliquots of 5 ml of the homogenates were mixed in 15-ml test tubes with 4 ml of distilled water and 1 ml of 50% trichloroacetic acid (TCA). The tubes were shaken intermittently for 15 minutes and centrifuged at 3000 x g for 10 minutes. Two milliliters of supernatant were mixed with 4 ml of 0.4 M Tris buffer at pH 8.9; 0.1 ml of DTNB was added and the sample was shaken. The absorbance was read within five minutes of addition of DTNB at 412 nM against a reagent blank (with no homogenates) in a spectrophotometer. The final NP-SH concentrations were calculated by comparing the absorbance of test with calibration curve prepared by using reduced glutathione solution and expressed as μmol per 100 mg of mucus.

The results obtained for the compounds of the invention and reference compounds GSH and GSNO are shown in Table 1 below.

Table 1.

| Treatment | Oral dose (mg/kg) | SH mucus content (μmol/100 mg) | Average of the ulcer score (mm) | Protection against ethanol (%) |
|---|---|---|---|---|
| Sham | 0 | 44 | 0 | |
| Ethanol | | 30 | 95 | |
| Example 7b | 2 | 75 | 38 | 60% |
| Example 8 | 2 | 49 | 50 | 47% |
| Example 16 | 2 | 23 | 41 | 57% |
| Example 22 | 2 | 77 | 24 | 75% |
| Example 25 | 2 | 52 | 53 | 44% |
| GSH | 2 | 79 | 59 | 38% |
| GSNO | 38 | 31 | 95 | 0% |

## Claims

**1.** A compound of formula (I):

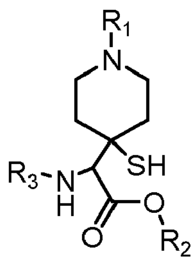

(I)

wherein

$R_1$ and $R_2$ are each independently selected from the group consisting of alkyl, alkenyl, cycloalkyl, alkynyl, aryl, aralkyl and heterocyclyl;

$R_3$ is selected from the group consisting of hydrogen, alkyl, cycloalkyl, alkenyl, alkynyl, heterocyclyl, $-C(=O)R_4$ and $-S(=O)_2R_4$, wherein $R_4$ is selected from the group consisting of hydrogen, alkyl, alkenyl, cycloalkyl, alkynyl, aryl, aralkyl and heterocyclyl;

wherein $R_4$ is optionally mono-, di-, or trisubstituted by a group independently selected from alkyl, alkyloxy, halogen and nitro;

or a pharmaceutically acceptable salt or stereoisomer thereof, for use as a medicament.

**2.** A compound for use according to claim 1, wherein $R_3$ is selected from the group consisting of $-C(=O)R_4$ and $-S(=O)_2R_4$, wherein $R_4$ is as defined in claim 1.

**3.** A compound for use according to claim 2, wherein $R_4$ is selected from the group consisting of hydrogen; alkyl optionally mono-, di-, or trisubstituted by halogen; aryl optionally mono-, di-, or trisubstituted by a group independently selected from alkyl, alkyloxy, halogen and nitro; and heterocyclyl.

**4.** A compound for use according to claim 3, wherein $R_4$ is selected from the group consisting of hydrogen; methyl optionally mono-, di- or trisubstituted by halogen; hexyl; phenyl optionally mono- or disubstituted by a group independently selected from halogen, methyl, methyloxy and nitro; and pyridyl.

**5.** A compound for use according to claim 4, wherein $R_4$ is selected from the group consisting of hydrogen; methyl optionally mono-, di- or trisubstituted by halogen; and phenyl optionally mono- or disubstituted by a group independently selected from halogen and nitro.

**6.** A compound for use according to any preceding claim, wherein $R_1$ and $R_2$ are each independently selected from the group consisting of alkyl and aralkyl.

**7.** A compound for use according to claim 6, wherein $R_1$ and $R_2$ are each independently selected from the group consisting of methyl, ethyl and benzyl.

**8.** A compound for use according to claim 7, wherein $R_1$ is selected from methyl and benzyl.

**9.** A compound for use according to any one of claims 7 to 8, wherein $R_2$ is ethyl.

**10.** A compound for use according to any preceding claim selected from the group consisting of:

- ethyl (R,S)-2-(4-mercapto-1-methylpiperidin-4-yl)-2-(phenyl sulfonamido)acetate;
- ethyl (R,S)-2-(4-mercapto-1-methylpiperidin-4-yl)-2-(4-nitrophenyl sulfonamido) acetate;
- ethyl (R,S)-2-(3-chlorophenylsulfonamido)-2-(4-mercapto-1-methyl piperidin-4-yl)acetate;
- ethyl (R,S)-2-(2-fluorophenylsulfonamido)-2-(4-mercapto-1-methyl piperidin-4-yl)acetate;
- ethyl (R,S)-2-(2,4-difluorophenylsulfonamido)-2-(4-mercapto-1-methyl piperidin-4-yl)acetate;
- ethyl (R,S)-2-acetamido-2-(1-benzyl-4-mercaptopiperidin-4-yl)acetate;
- ethyl (R,S)-2-(1-benzyl-4-mercaptopiperidin-4-yl)-2-formamidoacetate;

or a pharmaceutically acceptable salt or stereoisomer thereof.

**11.** A compound for use according to any preceding claim, wherein the medicament is for the treatment and/or prophylaxis of gastrointestinal ulcer.

**12.** A pharmaceutical composition comprising a compound of formula (I) as defined in any one of claims 1 to 10, or a pharmaceutically acceptable salt or stereoisomer thereof, and one or more pharmaceutically acceptable carriers.

**13.** A pharmaceutical composition according to claim 12 for oral administration.

Europäisches Patentamt
European Patent Office
Office européen des brevets

# EUROPEAN SEARCH REPORT

Application Number
EP 11 38 2377

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | EP 1 939 179 A1 (LACER SA [ES]) 2 July 2008 (2008-07-02) * claims 10, 16; synthesis examples * ----- | 1-13 | INV. A61K31/00 A61K31/445 A61K31/455 |
| A | LU S C ET AL: "ROLE OF GLUTATHIONE STATUS IN PROTECTION AGAINST ETHANOL-INDUCED GASTRIC LESIONS", PHARMACOLOGY (BASEL), vol. 38, no. 1, 1989, pages 57-60, XP009156428, ISSN: 0031-7012 * abstract * ----- | 1-13 | ADD. A61P1/04 |
| | | | TECHNICAL FIELDS SEARCHED (IPC) |
| | | | A61K |

1

The present search report has been drawn up for all claims

| Place of search | Date of completion of the search | Examiner |
|---|---|---|
| Munich | 10 February 2012 | Dahse, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 38 2377

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2012

| Patent document cited in search report | Publication date | Patent family member(s) | Publication date |
|---|---|---|---|
| EP 1939179 A1 | 02-07-2008 | AR 064542 A1 | 08-04-2009 |
| | | AU 2007341284 A1 | 10-07-2008 |
| | | CA 2673988 A1 | 10-07-2008 |
| | | CL 38342007 A1 | 11-07-2008 |
| | | CN 101600692 A | 09-12-2009 |
| | | CO 6190602 A2 | 19-08-2010 |
| | | EP 1939179 A1 | 02-07-2008 |
| | | EP 2125727 A1 | 02-12-2009 |
| | | ES 2307410 A1 | 16-11-2008 |
| | | JP 2010514732 A | 06-05-2010 |
| | | KR 20100090187 A | 13-08-2010 |
| | | PA 8764701 A1 | 18-12-2008 |
| | | PE 15542008 A1 | 23-12-2008 |
| | | US 2008161354 A1 | 03-07-2008 |
| | | UY 30849 A1 | 31-05-2008 |
| | | WO 2008080934 A1 | 10-07-2008 |
| | | ZA 200903645 A | 25-08-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008080534 A
- DE 2801849


### Non-patent literature cited in the description


- **SUTTON D. R. ; DONALDSON M.** *Gastroenterology,* 1975, vol. 69, 166-174
- **LANZA, F. L. et al.** *Amer. J. Gastroenterol.,* 1985, vol. 80, 767-769
- **LORENZ, R. L. et al.** *Lancet,* 1984, vol. 1, 1261-1264
- **BROOKS, F. P. et al.** Peptic Ulcer Disease: Contemporary Issues in Gastroenterology. 1985, 145-151
- **SZABO, S.** *Laboratory Investigation,* 1984, vol. 51, 121-147
- **THOMAS, J. M. et al.** *Clin. Gastroenterol.,* 1984, vol. 13, 501-541
- **GARNER, A. ; SCAND. J.** *Gastroenterol.,* 1986, vol. 21 (125), 203-210
- **MILLER, T. A.** *Amer. J. Physiol.,* 1983, vol. 245, G601-623
- **SZABO, S. et al.** *Science,* 1981, vol. 214, 200-202
- **LEUNG et al.** *Gastroenterology,* 1985, vol. 88, 1948-1953
- **SZABO et al.** *Gastroenterology,* 1985, vol. 88, 228-236
- **T.W. GREENE ; P.G.M. WUTS.** Protective Groups in Organic Synthesis. Wiley-Interscience, 454-493
- **NELSON C.F. YIM et al.** *J. Org. Chem.,* 1988, vol. 53, 4605-7
- **C. FREEMAN STANFIELD et al.** *Synthetic Communications,* 1988, vol. 18 (5), 531-43
- **NUNAMI KENICHI et al.** *J. Chem. Soc. Perkin Trans. 1,* 1979, vol. 9, 2224-9
- **E.W. MARTIN.** *Remington's Pharmaceutical Sciences*
- **C. FAULÍ I TRILLO.** Tratado de Farmacia Galénica. 1993
- **ROBERT A.** Cytoprotection by prostaglandins. *Gastroenterology,* 1979, vol. 77 (4), 761-767
- **SEDLAK J ; LINDSAY RH.** *Anal Biochem,* 1968, vol. 25, 192-5